# EUROPÄISCHE PATENTSCHRIFT

(11) **EP 2 528 639 B1**
(45) Veröffentlichungstag und Bekanntmachung des Hinweises auf die Patenterteilung: **03.06.2015**
(21) Anmeldenummer: 11701023.1
(22) Anmeldetag: 24.01.2011
(51) Int. Cl.: A61M 1/14, A61M 1/28

(54) **DIALYSEMASCHINE**
DIALYSIS MACHINE
MACHINE DE DIALYSE

(30) Priorität: 26.01.2010 DE 102010005745
(43) Veröffentlichungstag der Anmeldung: 05.12.2012
(73) Patentinhaber: Fresenius Medical Care Deutschland GmbH, 61346 Bad Homburg (DE)
(72) Erfinder: SEBESTA, Sven, 97421 Schweinfurt (DE); WERNICKE, Ulrich, 97531 Theres (DE)
(74) Vertreter: Laufhütte, Dieter
(86) Internationale Anmeldenummer: PCT/EP2011/000281
(87) Internationale Veröffentlichungsnummer: WO 2011/091975

(56) Entgegenhaltungen:
- EP-A2- 0 778 033
- US-A1- 2003 217 962
- US-A1- 2009 012 456

## Beschreibung

Die vorliegende Erfindung betrifft eine Dialysemaschine mit einer Ankoppelfläche zum Ankoppeln eines Fluidsystems, insbesondere zum Ankoppeln einer Kassette, und einem auf der Ankoppelfläche angeordneten Aktor zum Bewegen eines Aktorbereichs des Fluidsystems. Insbesondere kann es sich bei der Dialysemaschine dabei um eine Peritonealdialysemaschine oder um eine Hämodialysemaschine handeln.

Durch die Aufteilung des Dialysesystems in eine Dialysemaschine und ein Fluidsystem, welches üblicherweise als Einwegartikel ausgeführt ist, erfüllt das Fluidsystem hohe Ansprüche hinsichtlich der Sterilität. Zum Bewegen von Flüssigkeiten innerhalb des Fluidsystems und/oder zur Steuerung der Flüssigkeitsströme innerhalb des Fluidsystems werden dabei Aktoren der Dialysemaschine eingesetzt, welche auf entsprechende Aktorbereiche des Fluidsystems einwirken.

Bei Dialysemaschinen ist es oftmals notwendig, eine im Fluidsystem befindliche Flüssigkeit zu erwärmen. Insbesondere kann es sich dabei um Dialysat handeln, das auf Körpertemperatur erwärmt werden soll. Insbesondere bei der Peritonealdialyse müssen dabei große Mengen an frischem Dialysat erwärmt werden, bevor diese dem Patienten zugeführt werden. Dabei ist es bereits bekannt, die Flüssigkeit in einem Heizbeutel oder in einem Heizbereich einer Kassette zu erwärmen.

Aus der US 2009/0012456 A1 ist eine Dialysemaschine bekannt, welche eine Ankoppelfläche zum Ankoppeln eines Fluidsystems und insbesondere zum Ankoppeln einer Kassette, sowie einem auf der Ankoppelfläche angeordneten Aktor zum Bewegen eines Aktor-Bereichs des Fluidsystems aufweist. Um den Aktor zu beheizen, ist wenigstens ein Heizelement vorgesehen, wobei die Flüssigkeit im Aktor-Bereich des Fluidsystems über den Aktor erwärmt werden kann.

Aufgabe der vorliegenden Erfindung ist es, ausgehend von einem nächstliegenden Stand der Technik wie er in der Druckschrift US 2009/0012456 A1 offenbart ist, die Beheizbarkeit des Fluidsystems einer Dialysemschine zu verbessern.

Aufgabe der vorliegenden Erfindung ist es, die Beheizbarkeit des Fluidsystems einer Dialysemaschine zu verbessern.

Diese Aufgabe wird erfindungsgemäß durch eine Dialysemaschine gemäß Anspruch 1 gelöst. Die erfindungsgemäße Dialysemaschine umfasst dabei eine Ankoppelfläche zum Ankoppeln eines Fluidsystems, insbesondere zum Ankoppeln einer Kassette, und einen auf der Ankoppelfläche angeordneten Aktor zum Bewegen eines Aktorbereichs des Fluidsystems. Die erfindungsgemäße Dialysemaschine weist ein Heizelement zum Beheizen des Aktors auf, wobei die Flüssigkeit im Aktorbereich des Fluidsystems über den Aktor erwärmbar ist. Erfindungsgemäß ist es vorgesehen, dass der Aktor hydraulisch betätigt wird, und dass das Heizelement in einer Hydraulikpumpe oder in einer Hydraulikleitung angeordnet ist. Das Heizelement kann so die Hydraulikflüssigkeit des Aktores erwärmen. Über die Hydraulikpumpe kann dabei die erwärmte Hydraulikflüssigkeit zu einer flexiblen Membran, welche an dem Aktor-Bereich der Fluidwege anliegt, geführt werden. Somit kann auf eine einfache und kostengünstige Weise die Beheizbarkeit des Fluidsystems der Dialysemaschine verbessert werden.

Durch die vorliegende Erfindung kann die Fläche, welche zum Ankoppeln des Aktors an den Aktorbereich ohnehin benötigt wird, nun auch zum Erwärmen der Flüssigkeit im Fluidsystem herangezogen werden. Hierdurch wird die beheizbare Fläche des Fluidsystems erhöht, so dass das Fluidsystem kompakter aufgebaut werden kann. Zudem ergibt sich eine verbesserte Erwärmung der Flüssigkeit im Fluidsystem. Der Aktor ist dabei an den Aktorbereich des Fluidsystems angekoppelt und erwärmt so die Flüssigkeit, welche sich im Aktorbereich des Fluidsystems befindet bzw. durch diesen Bereich fließt. Das Heizelement kann dabei den Aktor direkt oder indirekt erwärmen.

Bei dem Aktorbereich des Fluidsystems handelt es sich dabei insbesondere um eine flexible Folie, welche durch einen auf der Ankoppelfläche der Dialysemaschine angeordneten Aktor bewegt werden kann. Insbesondere kann die flexible Folie dabei in eine Aussparung des Fluidsystems hineingedrückt und aus dieser herausgezogen werden. Der Aktor wirkt dann als ein Stößel, durch welchen die flexible Folie des Fluidsystems bewegt werden kann. Vorteilhafterweise ist der Aktorbereich dabei an einer Kassette angeordnet, welche an die Ankoppelfläche der Dialysema schine ankoppelbar ist. Eine solche Kassette erlaubt eine besonders kompakte Anordnung sowie eine einfache Bedienung.

Vorteilhafterweise handelt es sich bei dem Heizelement um ein aktives Heizelement, dessen Heizleistung zumindest durch Ein- und Ausschalten einstellbar ist. Insbesondere kann es sich dabei um ein elektrisches Heizelement handeln. Alternativ ist es jedoch auch denkbar, ein passives Heizelemente einzusetzen. Z.B. kann es sich dabei um ein Wärmeleitelement handeln, welches die Abwärme von weiteren Komponenten der Dialysemaschine sammelt und an den Aktor weiterleitet. Gegebenenfalls ist auch die Kombination eines passiven und eines aktiven Heizelements denkbar.

Weiterhin vorteilhafterweise ist erfindungsgemäß ein Temperatursensor zum Bestimmen der Temperatur des Aktors und/oder des Heizelements vorgesehen. Der Temperatursensor erlaubt so eine Regelung der Temperatur des Aktors.

Weiterhin vorteilhafterweise umfasst die Dialysemaschine eine Heizungssteuerung zur Ansteuerung des Heizelements. Vorteilhafterweise erfolgt die Ansteuerung des Heizelements dabei auf Grundlage des Ausgangssignals eines Temperatursensors. Die Heizungssteuerung kann dabei insbesondere auf Grundlage der Temperatur des Heizelements und/oder des Aktors und/oder der Flüssigkeit im Aktorbereich erfolgen.

Eine vorteilhafte Ausführungsform der vorliegenden Erfindung sieht vor, dass das Heizelement die Hydraulikflüssigkeit zum Antrieb des Aktors erwärmt. Hierdurch kann die Flüssigkeit im Aktorbereich des Fluidsystems über die Hydraulikflüssigkeit, welche den Aktor antreibt, erwärmt werden.

Weiterhin vorteilhafterweise umfasst der Aktor dabei eine flexible Membran, welche durch das Hydraulikfluid bewegt wird. Die flexible Membran arbeitet dabei im wesentlichen als ein Stößel, welcher den Aktorbereich des Fluidsystems bewegt.

Durch die vorliegende Erfindung wird so der Aktorbereich des Fluidsystems über das Hydraulikfluid erwärmt.

Vorteilhafterweise liegt dabei die flexible Membran an dem Aktorbereich der Fluidwege an. Vorteilhafterweise handelt es sich dabei auch bei dem Aktorbereich der Fluidwege um eine flexible Folie, welche an der flexiblen Membran anliegt.

Vorteilhafterweise ist vorgesehen, dass das Heizelement elektrisch von der Hydraulikflüssigkeit isoliert ist. Hierdurch ergibt sich eine erste Isolation zwischen der Dialysemaschine und dem Fluidsystem.

Weiterhin vorteilhafterweise ist vorgesehen, dass die flexible Membran elektrisch isolierend ausgeführt ist. Somit ergibt sich durch die flexible Membran des Aktors eine zweite Isolierung zwischen Dialysemaschine und Fluidsystem.

Die flexible Membran des Aktors kann dabei insbesondere als eine Silikonmatte ausgeführt sein. Vorteilhafterweise ist die Silikonmatte dabei gut wärmeleitend aber elektrisch isolierend ausgeführt.

Weiterhin vorteilhafterweise ist vorgesehen, dass der Temperatursensor zum Bestimmen der Temperatur des Aktors die Temperatur des Hydraulikfluids misst. Hierdurch kann das Heizelement so angesteuert werden, dass die Temperatur des Hydraulikfluids geregelt wird.

Der erfindungsgemäße Aktor kann sowohl zum Bewegen von Flüssigkeiten als auch zur Ansteuerung von Fluidwegen im Fluidsystem eingesetzt werden. In einer bevorzugten Ausführung handelt es sich bei dem Aktor dabei um einen Pumpaktor zum Bewegen einer Pumpmembran des Fluidsystems. Vorteilhafterweise bewegt der Pumpaktor dabei eine Pumpmembran einer Pumpkammer des Fluidsystems, wodurch Flüssigkeit durch das Fluidsystem bewegbar ist. Dabei steht eine relativ große Ankoppelfläche zur Verfügung, über welche die Flüssigkeit im Pumpbereich des Fluidsystems erwärmt werden kann. Zudem wird die Flüssigkeit während des Pumpens bewegt.

Weiterhin vorteilhafterweise umfasst die erfindungsgemäße Dialysemaschine ein weiteres Heizelement, welches mit einem unbewegten Heizbereich des Fluidsystems koppelbar ist. Neben der erfindungsgemäßen Möglichkeit der Beheizung der Flüssigkeit über den Aktor der Dialysemaschine steht damit noch ein weiteres Heizelement zur Verfügung. Bei diesem Heizelement kann es sich dabei um ein bereits bekanntes Heizelement, welches z. B. an einen Heizbereich einer Kassette oder an einen Heizbeutel des Fluidsystems koppelt, handeln.

Vorteilhafterweise wird dabei der erfindungsgemäß erwärmbare Aktor eingesetzt, um das zum Heizbereich fließende Fluid vorzuheizen. Insbesondere bei sehr kaltem Fluid kann so der erfindungsgemäße Aktor den Heizbereich entlasten, in dem er die Flüssigkeit im Fluidsystem auf eine Mindesttemperatur erwärmt.

Vorteilhafterweise wird das Heizelement des Aktors dabei über die Heizungssteuerung der Dialysemaschine, welche auch das weitere Heizelement ansteuert, gesteuert. Die Ansteuerung erfolgt dabei vorteilhafterweise auf Grundlage eines Sensors, welcher die Temperatur der Flüssigkeit im Fluidsystem misst.

Die vorliegende Erfindung umfasst weiterhin ein System aus einer Dialysemaschine, wie sie oben beschrieben wurde, und einem Fluidsystem. Insbesondere handelt es sich bei dem Fluidsystem dabei um eine Kassette, welche an der Ankoppelfläche der Dialysemaschine ankoppelbar ist. Vorteilhafterweise ist dabei ein Aktorbereich der Kassette über den erfindungsgemäßen Aktor erwärmbar.

Die vorliegende Erfindung umfasst weiterhin ein Verfahren zum Betrieb einer Dialysemaschine, mit den Schritten: Ankoppeln eines Fluidsystems, insbesondere einer Kassette, an einer Ankoppelfläche einer Dialysemaschine; Bewegen eines Aktorbereichs des Fluidsystems durch einen auf der Ankoppelfläche angeordneten hydraulisch betätigbaren Aktor, wobei ein Heizelement zum Beheizen des Aktors in einer Hydraulikpumpe oder in einer Hydraulikleitung angeordnet ist; und Erwärmen einer im Aktorbereich des Fluidsystems befindlichen Flüssigkeit über den Aktor. Durch das erfindungsgemäße Verfahren ergeben sich die gleichen Vorteile, welche bereits oben hinsichtlich der Dialysemaschine beschrieben wurden. Insbesondere kann dabei der Aktorbereich des Fluidsystems genutzt werden, um Flüssigkeit im Fluidsystem zu erwärmen.

Vorteilhafterweise erfolgt das Verfahren dabei so, wie dies bereits oben im Hinblick auf die Dialysemaschine dargestellt wurde. Insbesondere handelt es sich bei dem Verfahren dabei um ein Verfahren zum Betrieb einer Dialysemaschine, wie sie oben dargestellt wurde.

Die vorliegende Erfindung wird nun anhand von Ausführungsbeispielen sowie Zeichnungen näher dargestellt. Dabei zeigen:

Dabei zeigen:
- Fig. 1: drei Diagramme, welche typische Verläufe einer automatischen Peritonealdialysebehandlung zeigen,
- Fig. 2: eine Prinzipdarstellung eines Peritonealdialysesystems,
- Fig. 3: eine Prinzipdarstellung der Aufteilung des Peritonealdialysesystems in eine Dialysemaschine und ein Fluidsystem,
- Fig. 4: ein erstes Ausführungsbeispiel einer Kassette,
- Fig. 5: ein zweites Ausführungsbeispiel einer Kassette,
- Fig. 6: eine perspektivische Ansicht eines ersten Ausführungsbeispiels einer Dialysemaschine,
- Fig. 7: ein Flussdiagramm eines ersten Ausführungsbeispiels eines Peritonealdialysesystems,
- Fig. 8: eine perspektivische Ansicht eines zweiten Ausführungsbeispiels einer Dialysemaschine,
- Fig. 9: ein Flussdiagramm eines zweiten Ausführungsbeispiels eines Peritonealdialysesystems,
- Fig. 10: das Ankoppeln der Kassette beim zweiten Ausführungsbeispiels eines Peritonealdialysesystems,
- Fig. 11: ein erstes Ausführungsbeispiel eines Pumpaktors,
- Fig. 12: das Ankoppeln eines Pumpbereichs der Kassette an einen Pumpaktor,
- Fig. 13: eine Prinzipdarstellung des Aufbaus eines Ausführungsbeispiels einer Steuerung, und
- Figur 14: ein erstes Ausführungsbeispiel eines beheizbaren Aktors gemäß der vorliegenden Erfindung.

Im folgenden soll die Funktion einer Dialysemaschine, bei welcher die vorliegende Erfindung zum Einsatz kommt, zunächst allgemein beschrieben werden. Bei der Dialysemaschine handelt es sich dabei im Ausführungsbeispiel um eine Peritonealdialysemaschine. Die unten beschriebenen Komponenten können jedoch in gleicher oder ähnlicher Weise auch für eine Hämodialysemaschine eingesetzt werden.

Die Peritonealdialyse ist eine Variante der künstlichen Blutwäsche, bei welcher das gut durchblutete Bauchfell (Peritonium) des Patienten als körpereigene Filtermembran verwendet wird. Hierfür wird über einen Katheter Dialysat in die Bauchhöhle eingeführt. Nach dem Prinzip der Osmose diffundieren nun Harnbestandteile des Blutes durch das Bauchfell in das in der Bauchhöhle befindliche Dialysat. Nach einer gewissen Verweilzeit wird das Dialysat mit den Harnbestandteilen wieder aus der Bauchhöhle ausgelassen.

Bei der automatischen Peritonealdialyse steuert und überwacht eine Dialysemaschine die Einführung des frischen Dialysats in die Bauchhöhle und das Auslassen des verbrauchten Dialysats Eine solche Dialysemaschine, auch Cycler genannt, befüllt und entleert die Bauchhöhle dabei üblicherweise mehrmals über Nacht, d.h. während der Patient schläft.

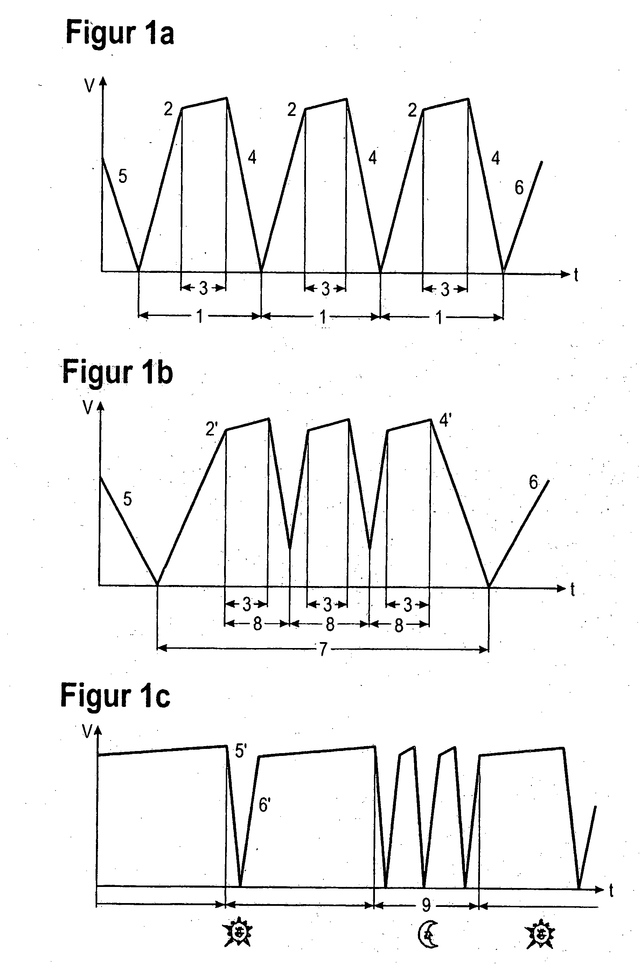

In Figuren 1a bis 1c sind drei unterschiedliche Verfahrensabläufe gezeigt, wie sie von einer Dialysemaschine durchgeführt werden. Einer oder mehrerer dieser Verfahrensabläufe ist dabei üblicherweise in der Steuerung der Dialysemaschine hinterlegt. Dabei ist es üblicherweise möglich, die abgespeicherten Verfahrensabläufe an den Patienten anzupassen.

Bei den Figuren 1a bis 1c ist jeweils die in der Bauchhöhle des Patienten befindliche Dialysatmenge V über die Zeit t aufgezeichnet. Figur 1 a zeigt dabei den Verlauf einer normalen automatischen Peritonealdialysebehandlung über Nacht. Zu Beginn der Behandlung erfolgt dabei zunächst ein initialer Auslauf 5, durch welchen Dialysat, das über den Tag in der Bauchhöhle des Patienten belassen worden war, entnommen wird. Daraufhin folgen mehrere Behandlungszyklen 1, in Figur 1a drei aufeinander folgende Behandlungszyklen 1. Jeder Behandlungszyklus besteht dabei aus einer Einlaufphase 2, einer Verweilphase 3 und einer Auslaufphase 4. Während der Einlaufphase 2 wird dabei ein gewisses Volumen an frischer Dialyseflüssigkeit in die Bauchhöhle des Patienten eingelassen. Die maximal zulässige Dialysatmenge beträgt dabei je nach Patienten zwischen ca. 1,5 und 3 l. Das frische Dialysat verbleibt nun über eine gewisse Verweilzeit 3 in der Bauchhöhle. Typischerweise dauert die Verweilphase dabei einige Stunden. Daraufhin wird das nun verbrauchte Dialysat in der Auslaufphase 4 wieder aus der Bauchhöhle herausgelassen. Daraufhin startet ein neuer Behandlungszyklus. Die Behandlung wird mit einem letzten Einlauf 6 abgeschlossen, durch welchen eine gewisse Menge an frischem Dialysat in die Bauchhöhle des Patienten eingeführt wird. Diese verbleibt dann über den Tag in der Bauchhöhle des Patienten.

Die einzelnen Behandlungszyklen 1, welche über Nacht erfolgen, werden dabei automatisch von der Steuerung der Dialysemaschine angesteuert. Der initiale Auslauf und der letzte Einlauf können ebenfalls automatisch von der Dialysemaschine angesteuert werden. Alternativ werden diese manuell von einer Bedienperson oder vom Patienten aktiviert.

In Figur 1b ist eine sogenannte Tidalbehandlung gezeigt. Auch diese beginnt mit einem initialen Auslauf 5 und endet mit einem letzten Einlauf 6. Weiterhin ist ein Basiszyklus 7 vorgesehen, welcher sich in mehrere Tidalzyklen 8 aufteilt. Dabei ist zunächst eine Basiseinlaufphase 2' vorgesehen. Nach der Verweilphase 3 wird jedoch nicht mehr das komplette Dialysatvolumen aus der Bauchhöhle entnommen, sondern nur eine gewisse Teilmenge des in der Bauchhöhle befindlichen Dialysats. Dieses wird dann durch ein entsprechendes Volumen an frischem Dialysat ersetzt. Nach einem erneuten Verweilzyklus kann eine weitere Tidalentnahme erfolgen, bei welcher nicht das gesamte im Bauchraum befindliche Dialysat entfernt wird. Zum Ende des Basiszyklus 7 erfolgt eine Basisauslaufphase 4', bei welcher nun das gesamte Dialysat entnommen wird. In Figur 1b ist dabei lediglich ein Basiszyklus 1 dargestellt. Alternativ können jedoch auch mehrere Basiszyklen vorgesehen sein.

In Figur 1c ist der Verlauf einer Peritonealdialysebehandlung mit einer sogenannten PD-Plus-Behandlung gezeigt. Dabei erfolgt während der Nacht 9 eine übliche Peritonealdialysebehandlung, welche z. B. gemäß den Figuren 1 a oder 1 b durchgeführt werden kann. Weiterhin ist jedoch während des Tages eine zusätzliche PD-Plus-Behandlung vorgesehen, bei welcher in einer Auslaufphase 5' das verbrauchte Dialysat entnommen und in einer Einlaufphase 6' durch frisches Dialysat ersetzt wird. Bei der PD-Plus-Behandlung wird damit eine normale nächtliche Peritonealdialysebehandlung mit einem oder mehreren zusätzlichen Behandlungszyklen während des Tages kombiniert. Der Verlauf der nächtlichen Behandlung wird dabei wie üblich automatisch durch die Dialysemaschine durchgeführt. Die Behandlungszyklen während des Tages werden ebenfalls über die Maschine durchgeführt und überwacht.

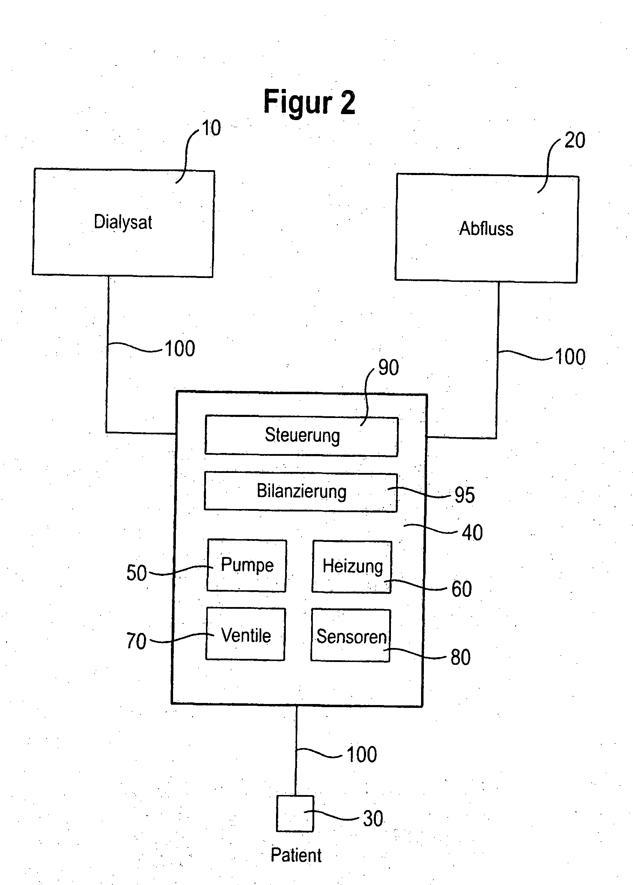

In Figur 2 ist nun der Aufbau eines typischen Peritonealdialysesystems schematisch dargestellt. Das Peritonealdialysesystem umfasst dabei einen Behälter 10 mit frischem Dialysat und einen Abfluss 20 für gebrauchtes Dialysat. Weiterhin ist ein Konnektor 30 vorgesehen, welcher an einen Katheter des Patienten angeschlossen werden kann, um entweder frisches Dialysat in die Bauchhöhle des Patienten einzuführen oder gebrauchtes Dialysat aus der Bauchhöhle abzuführen. Der Behälter 10 mit frischem Dialysat, der Abfluss 20 für gebrauchtes Dialysat und der Konnektor 30 zum Patienten sind dabei über Fluidwege 100 miteinander verbunden und bilden zusammen mit diesen das Fluidsystem des Peritonealdialysesystems.

Zur Durchführung der Peritonealdialysebehandlung ist eine Dialysemaschine 40, auch Cycler genannt, vorgesehen. Die Dialysemaschine 40 umfasst dabei folgende Hauptkomponenten:
- Eine Pumpe 50, welche zum Transport der Flüssigkeiten eingesetzt wird. Die Pumpe 50 befördert dabei das frische Dialysat vom Behälter 10 zum Konnektor 30. Weiterhin kann die Pumpe 50 das verbrauchte Dialysat vom Konnektor 30 zum Abfluss 20 transportieren.
- Ventile 70, welche zur Steuerung der Flüssigkeitsströme eingesetzt werden. Die Ventile 70 öffnen und schließen die Fluidwege 100, um so die entsprechenden Fluidverbindungen zwischen dem Behälter 10, dem Konnektor 30 und dem Abfluss 20 herzustellen.
- Eine Heizung 60, welche das frische Dialysat auf eine Temperatur von ca. 37°C bringt, bevor dieses dem Patienten zugeführt wird. Da bei der Peritonealdialyse relativ große Mengen an Dialysat direkt in die Bauchhöhle des Patienten eingeführt werden, ist die Heizung 60 nötig, um den Patienten nicht zu unterkühlen und um ein unangenehmes Gefühl durch zu kaltes Dialysat zu vermeiden.
- Sensoren 80, über welche der ordnungsgemäße Ablauf der Behandlung überwacht und/oder gesteuert werden kann. Insbesondere können dabei Temperatursensoren zum Einsatz kommen. Weiterhin können gegebenenfalls Drucksensoren zum Einsatz kommen.

Alle Komponenten der Dialysemaschine 40 werden dabei über eine Steuerung 90 angesteuert. Die Steuerung 90 steuert dabei insbesondere die Pumpe 50, die Heizung 60 und die Ventile 70 auf Grundlage der Daten der Sensoren 80 an Die Steuerung 90 sorgt dabei für den automatischen Ablauf der Peritonealdialyse. Als wichtige Komponente umfasst die Steuerung 90 dabei eine Bilanzierung 95, welche die dem Patienten zugegebenen und entnommenen Flüssigkeitsmengen bilanziert. Die Bilanzierung verhindert dabei, dass dem Patienten zuviel Flüssigkeit zugegeben oder zuviel Flüssigkeit entnommen wird.

Die Bilanzierung 95 kann dabei allein auf Grundlage der Ansteuerdaten und/oder der Sensordaten für die Pumpe 50 erfolgen. Alternativ kann die Bilanzierung auch über separat vorgesehene Bilanzierkammern erfolgen. Ebenso ist es möglich, zur Bilanzierung eine Waage einzusetzen. Eine solche Waage wiegt beispielsweise das Gewicht des Behälters 10 mit frischem Dialysat und/oder eines Behälters 20 mit gebrauchtem Dialysat.

Da bei der Peritonealdialyse das Dialysat dem Patienten direkt in die Bauchhöhle verabreicht wird, ist auf äußerste Sterilität zu achten. Daher werden die Fluidwege bzw. das Fluidsystem, welches mit dem frischen und/oder dem gebrauchten Dialysat in Kontakt kommt, üblicherweise als Einwegteil ausgeführt. Insbesondere werden die Fluidwege bzw. das Fluidsystem dabei als Kunststoffteile ausgeführt. Diese können so in einer sterilen Umverpackung angeliefert und erst kurz vor der Behandlung ausgepackt werden.

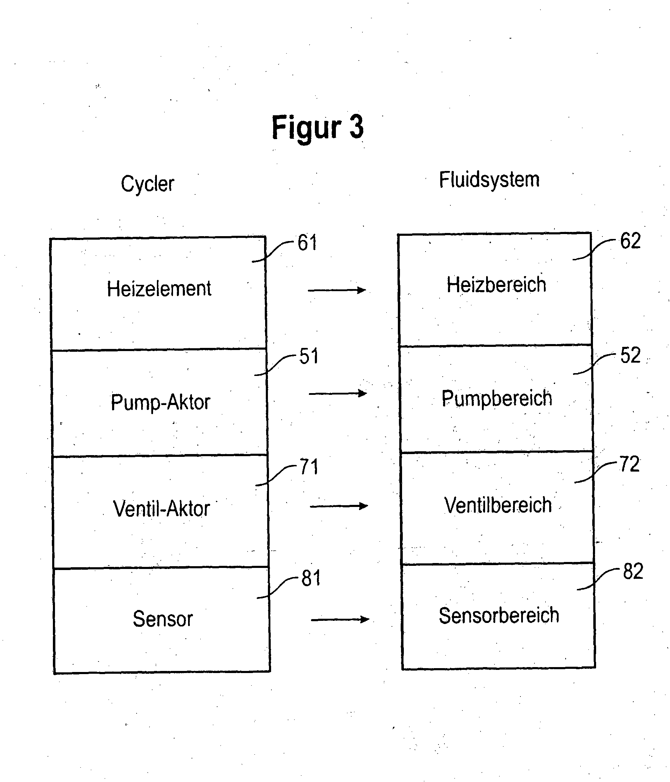

Um dennoch eine Steuerung der Peritonealdialyse durch die Dialysemaschine 40 zu ermöglichen, muß das Fluidsystem an die Dialysemaschine 40 angekoppelt werden. In Figur 3 ist dabei schematisch dargestellt, wie einzelne Elemente der Dialysemaschine 40 an entsprechende Bereiche des Fluidsystems angekoppelt werden.

Die Dialysemaschine 40 weist dabei ein Heizelement 61 auf. Dieses muss an einen entsprechenden Heizbereich 62 des Fluidsytems angekoppelt werden. Die Ankopplung ermöglicht dabei die Übertragung von Wärmeenergie von dem Heizelement 61 an das im Heizbereich 62 befindliche Dialysat.

Die Dialysemaschine 40 weist weiterhin einen oder mehrere Pumpaktoren 51 auf, welche mit einem Pumpbereich 52 des Fluidsystems gekoppelt werden. Die Pumpaktoren 51 erzeugen dabei eine Pumpkraft, welche auf den Pumpbereich 52 übertragen wird. Hierdurch kann die im Pumpbereich 52 befindliche Flüssigkeit entlang der Fluidwege bewegt werden.

Weiterhin weist die Dialysemaschine einen oder mehrere Ventilaktoren 71 auf. Diese erzeugen eine Schließbewegung, welche auf entsprechende Ventilbereiche 72 der Fluidwege übertragen wird. Hierdurch können die Ventilbereiche 72 der Fluidwege entsprechend geschlossen bzw. geöffnet werden.

Weiterhin weist die Dialysemaschine einen oder mehrere Sensoren 81 auf. Diese werden an einen entsprechenden Sensorbereich 82 des Fluidsystems angekoppelt. Hierdurch können die Sensoren 81 gewisse Eigenschaften des Dialysats messen. Insbesondere kann hierdurch die Temperatur des Dialysats gemessen werden. Weiterhin kann vorgesehen sein, dass der Druck in dem Fluidsystem bestimmt wird.

Selbstverständlich weist die Dialysemaschine gegebenenfalls noch weitere Aktoren und/oder Sensoren auf, welche nicht mit den Fluidwegen gekoppelt werden müssen.

Die einzelnen Komponenten eines Peritonealdialysesystems sollen nun im folgenden näher anhand von Ausführungsbeispielen dargestellt werden.

### 1. Fluidsystem

### 1.1 Dialysatbehälter

Frisches Dialysat wird üblicherweise in Kunststoffbeuteln zur Verfügung gestellt. Solche Kunststoffbeutel weisen üblicherweise zwei Lagen von Kunststofffolie auf, welche in einem Randbereich miteinander verschweißt sind und so einen Behälter bilden, welcher mit frischem Dialysat befüllt ist. An diesen Behälter ist üblicherweise ein Schlauchelement angeschweißt, durch welches das Dialysat aus dem Beutel entnommen werden kann. An dem Schlauchelement ist üblicherweise ein Konnektor angeordnet, über welchen der Dialysatbehälter mit den übrigen Fluidwegen verbunden werden kann. Weiterhin weist der Beutel üblicherweise an der dem Schlauch gegenüberliegenden Seite eine Aussparung oder Öse auf, über welche der Beutel an einen Haken aufgehängt werden kann. Hierdurch kann sichergestellt werden, dass das Dialysat problemlos aus dem Beutel abfließt.

Das Dialysat besteht üblicherweise aus einem Puffer, einem Osmotikum und Elektrolyten. Als Puffer kann dabei z. B. Bicarbonat eingesetzt werden. Als Osmotikum wird üblicherweise Glucose eingesetzt. Alternativ können auch Glucosepolymere oder Glucosepolymerderivate eingesetzt werden. Die Elektrolyte umfassen üblicherweise Kalzium und Natrium.

Das Dialysat kann dabei hitzesterilisiert werden. Dies erfolgt vorteilhafterweise, nachdem das Dialysat in den Beutel gefüllt wurde. Hierdurch werden sowohl das Dialysat als auch der Beutel hitzesterilisiert. Dabei wird der gefüllte Beutel üblicherweise zunächst in eine Umverpackung verpackt, woraufhin das gesamte System sterilisiert wird.

Da die fertige Dialysatlösung je nach Inhaltsstoffen oft nicht hitzesterilisiert werden kann oder nicht lange gelagert werden kann, kann vorgesehen sein, einzelne Komponenten des Dialysats getrennt zu lagern und erst kurz vor der Behandlung zusammenzuführen. Eine erste Einzellösung umfasst dabei üblicherweise den Puffer, während eine zweite Einzellösung Glucose und Elektrolyte umfasst. Gegebenenfalls können auch mehr als zwei Einzellösungen und damit mehr als zwei Bereiche in einem Beutel vorgesehen sein. Dabei kann ein Mehrfachkammerbeutel, insbesondere ein Doppelkammerbeutel vorgesehen sein, welcher mehrere getrennte Bereiche zur Lagerung der Einzellösungen aufweist. Diese Bereiche sind durch ein Verbindungselement getrennt, welches mechanisch geöffnet werden kann, um die Einzelflüssigkeiten miteinander zu vermischen. Insbesondere kann dabei eine sogenannte Peel-Naht zwischen den beiden Bereichen des Beutels vorgesehen sein, welche sich bei Anwendung eines bestimmten Druckes auf mindestens einen der Bereiche des Beutels öffnet.

Da während einer nächtlichen Peritonealdialysebehandlung relativ große Mengen an Dialysat verbraucht werden, werden üblicherweise mehrere Dialysatbehälter parallel eingesetzt. Diese werden über entsprechende Konnektoren mit den Fluidwegen verbunden und können durch eine entsprechende Schaltung der Ventile zum Befüllen des Patienten herangezogen werden.

### 1.2 Abfluss

Zum Entsorgen der verbrauchten Dialysierflüssigkeit kann diese entweder sofort in die Kanalisation abgeführt oder zunächst in einem Abflussbehälter gesammelt werden. Als Abflussbehälter wird dabei üblicherweise ebenfalls ein Beutel eingesetzt. Dieser ist vor Beginn der Behandlung leer und kann so das verbrauchte Dialysat aufnehmen. Der Beutel kann dann nach Beendigung der Behandlung entsprechend entsorgt werden.

### 1.3 Kassette

Wie bereits eingangs beschrieben, weist das Fluidsystem eine Mehrzahl von Bereichen auf, in welchen die Dialysemaschine auf das Fluidsystem einwirken muss. Hierfür muss das Fluidsystem an die Dialysemaschine angekoppelt werden.

Um das Ankoppeln der Fluidwege an die Dialysemaschine und die Einwirkung der entsprechenden Elemente der Dialysemaschine auf die Fluidwege zu vereinfachen, werden Kassetten eingesetzt. In einer solchen Kassette sind mehrere Bereiche, in weichen die Dialysemaschine auf die Fluidwege einwirkt, gemeinsam angeordnet. Hierfür weist eine Kassette üblicherweise ein Hartteil aus Kunststoff auf, in welches zu einer Seite hin offene Kammern als Fluidwege eingebracht sind. Diese Kammern werden von einer flexiblen Kunststofffolie bedeckt, welche für die Ankopplung an die Dialysemaschine sorgt. Die flexible Kunststofffolie ist dabei üblicherweise in einem Randbereich mit dem Hartteil verschweißt. Die Kassette wird mit einer Ankoppelfläche der Dialysemaschine verpresst, so dass die Aktoren und/oder Sensoren der Dialysemaschine, mit entsprechenden Bereichen der Kassette in Kontakt kommen.

Die Kassette weist weiterhin Anschlüsse zum Anschluss des Dialysatbehälters 10, des Konnektors 30 sowie des Ablaufs 20 auf.

Eine Kassette umfasst dabei üblicherweise zumindest einen Pumpbereich und einen oder mehrere Ventilbereiche. Über die Kassette kann so der Flüssigkeitstransport durch das Fluidsystem,gesteuert werden. Weiterhin kann die Kassette Sensorbereiche aufweisen, welche eine einfache Ankopplung von Sensoren der Dialysemaschine an das Fluidsystem ermöglichen. Gegebenenfalls kann die Kassette weiterhin einen oder mehrere Heizbereiche aufweisen, welche an entsprechende Heizelemente der Dialysemaschine ankoppelbar sind.

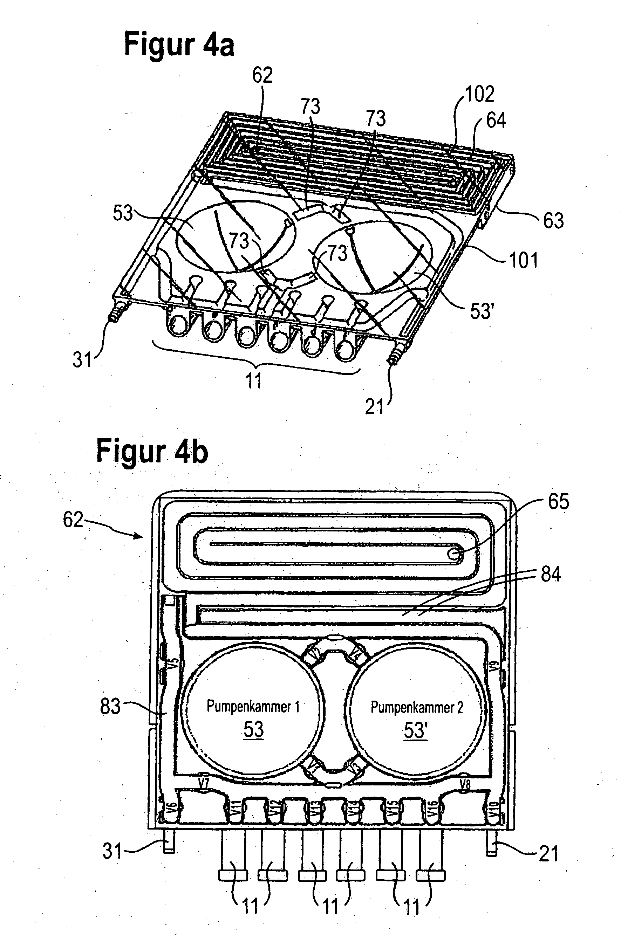

In Figuren 4a und 4b ist ein erstes Ausführungsbeispiel einer Kassette dargestellt. Diese weist ein Hartteil 101 aus Kunststoff auf, in welchem die Fluidwege und Ankopplungsbereiche als entsprechende Aussparungen, Kammern und Kanäle eingebracht sind. Das Hartteil kann dabei z. B. als Spritzgussteil oder als Tiefziehteil jefertigt werden. Die Ankoppelebene des Hartteils 101 wird von einer flexiblen Folie 102 bedeckt, welche in einem Randbereich mit dem Hartteil verschweißt ist. Durch das Verpressen der Kassette mit einer Ankoppelfläche der Dialysemaschine wird die flexible Folie 102 mit dem Hartteil verpresst. Durch das Verpressen der flexiblen Folie mit den Stegbereichen des Hartteils werden die Fluidwege innerhalb der Kassette fluiddicht voneinander getrennt.

Die Kassette weist Anschlüsse zum Anschluss der Kassette an die übrigen Fluidwege auf. Zum einen ist ein Anschluss 21 zum Anschluss an den Abfluss 20 sowie ein Anschluss 31 zum Anschluss an den Konnektor 30 vorgesehen. An diesen Anschlüssen können entsprechende Schlauchelemente vorgesehen sein, welche in Figur 4a nicht dargestellt sind. Weiterhin weist die Kassette eine Mehrzahl von Anschlüssen 11 zum Anschluss von Dialysatbehältern 10 auf. Die Anschlüsse 11 sind dabei im ersten Ausführungsbeispiel als Konnektoren ausgeführt, an welche entsprechende Konnektorelemente angeschlossen werden können.

Die Anschlüsse stehen jeweils mit Fluidwegen innerhalb der Kassette in Verbindung. In diesen Fluidwegen sind Ventilbereiche vorgesehen. In diesen Ventilbereichen kann die flexible Folie 102 über maschinenseitige Ventilaktoren so in das Hartteil 101 gedrückt werden, dass der entsprechende Fluidweg versperrt ist. Die Kassette weist dabei zunächst einmal für jeden Anschluss ein entsprechendes Ventil auf, über welches dieser Anschluss geöffnet bzw. geschlossen werden kann. Dem Anschluss 21 für den Abfluss 20 ist dabei das Ventil V10 zugeordnet, dem Anschluss 31 für den Patientenkonnektor 30 das Ventil V6. Den Anschlüssen 11 für die Dialysatbehälter 10 sind die Ventile V11 bis V16 zugeordnet.

Weiterhin sind in der Kassette Pumpenkammern 53 und 53' vorgesehen, welche durch entsprechende Pumpaktoren der Dialysemaschine betätigt werden können. Bei den Pumpenkammern 53 und 53' handelt es sich dabei um konkave Aussparungen in dem Hartteil 101, welche von der flexiblen Folie 102 bedeckt werden. Durch Pumpaktoren der Dialysemaschine kann die Folie nun in die Pumpenkammern 53 und 53' hineingedrückt bzw. wieder aus diesen Pumpenkammern herausgezogen werden. Hierdurch kann, im Zusammenspiel mit den Ventilen V1 bis V4, welche die Zugänge und Abläufe der Pumpenkammern 53 und 53' schalten und in Fig. 4a mit dem Bezugszeichen 73 bezeichnet wurden, ein Pumpstrom durch die Kassette erzeugt werden. Die Pumpkammern sind dabei über entsprechende Ventilschaltungen mit allen Anschlüssen der Kassette verbindbar.

Weiterhin ist ein Heizbereich 62 in die Kassette integriert. In diesem Bereich wird die Kassette mit Heizelementen der Dialysemaschine in Kontakt gebracht, welche das durch diesen Bereich der Kassette fließende Dialysat erwärmen. Der Heizbereich 62 weist dabei einen Kanal für das Dialysat auf, welcher sich spiralförmig über den Heizbereich 62 erstreckt. Der Kanal wird dabei durch Stege 64 des Hartteils gebildet, welche von der flexiblen Folie 102 abgedeckt sind.

Der Heizbereich 62 ist dabei auf beiden Seiten der Kassette vorgesehen. Hierfür ist auch auf der Unterseite 63 der Kassette im Heizbereich eine flexible Folie am Hartteil angeordnet. Die flexible Folie ist dabei ebenfalls in einem Randbereich mit dem Hartteil verschweißt. Auf der Unterseite ist ebenfalls ein Kanal angeordnet, durch welchen das Dialysat fließt. Die Kanäle auf der Unterseite und der Oberseite werden dabei durch eine mittlere Platte des Hartteils gebildet, welche die Oberseite von der Unterseite trennt, und auf welcher nach unten und nach oben Stege vorgesehen sind, welche die Kanalwandungen bilden. Dabei fließt das Dialysat zunächst spiralförmig auf der Oberseite bis zum Durchbruch 65 durch die mittlere Platte, von wo aus das Dialysat auf der Unterseite durch den entsprechenden Kanal zurückfließt. Durch den auf der Ober- und der Unterseite vorgesehenen Heizbereich kann die Heizfläche, welche zum Aufheizen der Flüssigkeit zur Verfügung steht, entsprechend vergrößert werden. Selbstverständlich ist aber auch eine Ausführungsform der Kassette möglich, bei welcher nur auf einer Seite der Kassette ein Heizbereich angeordnet ist.

Weiterhin sind Ausführungsformen der Kassette möglich, bei welcher ein Heizelement in die Kassette integriert ist. Insbesondere kann dabei ein elektrisches Heizelement wie z.B. Heizwendel in das Hartteil der Kassette eingegossen sein. Hierdurch kann auf ein maschinenseitiges Heizelement verzichtet werden und die Durchflussheizung in die Kassette integriert werden. Dabei sind an der Kassette elektrische Kontakte zur Konnektierung des elektrischen Heizelements angeordnet.

Die Kassette weist weiterhin Sensorbereiche 83 und 84 auf, durch welche Temperatursensoren der Dialysemaschine an die Kassette gekoppelt werden können. Die Temperatursensoren liegen dabei auf der flexiblen Folie 102 auf und können so die Temperatur der durch den darunter liegenden Kanal fließenden Flüssigkeit messen. Dabei sind am Eingang des Heizbereiches zwei Temperatursensoren 84 vorgesehen. Am patientenseitigen Ausgang ist ein Temperatursensor 83 vorgesehen, über welchen die Temperatur des zum Patienten gepumpten Dialysats gemessen werden kann.

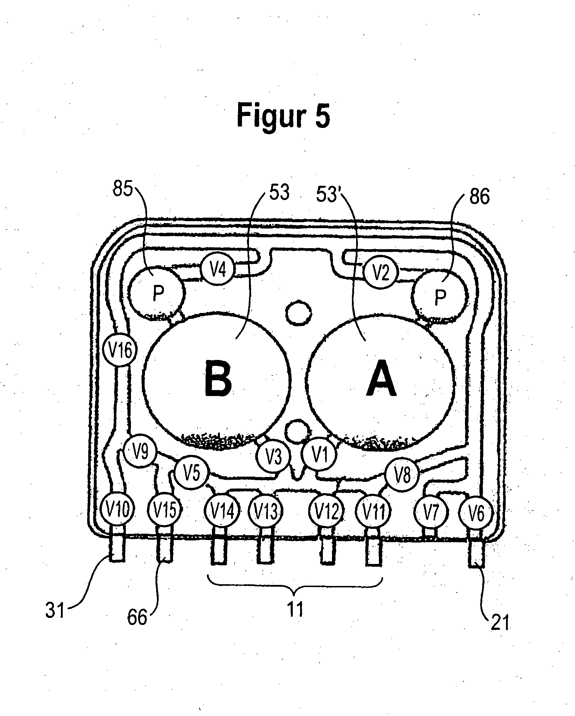

In Figur 5 ist ein zweites Ausführungsbeispiel für eine Kassette gezeigt. Die Kassette entspricht dabei in ihrer Ausführung im wesentlichen dem ersten Ausführungsbeispiel, umfasst jedoch keinen Heizbereich. Bei Einsatz dieser Kassette erfolgt die Heizung daher nicht wie beim ersten Ausführungsbeispiel gezeigt über einen in die Kassette integrierten Heizbereich, sondern z.B. über einen Heizbeutel, welcher auf eine Heizplatte der Dialysemaschine gelegt wird.

Das in Figur 5 gezeigte zweite Ausführungsbeispiel einer Kassette weist wiederum Fluidwege auf, welche über Ventilbereiche, die hier ebenfalls von V1 bis V16 durchnumeriert sind, geöffnet und geschlossen werden können. Weiterhin weist die Kassette Anschlüsse zum Anschluss an weitere Komponenten des Fluidsystems auf. Dabei ist wiederum der Anschluss 21 zum Anschluss an den Abfluss 20, sowie der Anschluss 31 zum Anschluss an den Konnektor 30 zum Patienten vorgesehen. Weiterhin sind Anschlüsse 11 zum Anschluss von Dialysatbehältern 10 vorgesehen.

Im Unterschied zum ersten Ausführungsbeispiel weist die im zweiten Ausführungsbeispiel gezeigte Kassette einen weiteren Anschluss 66 zum Anschluss eines Heizbeutels auf. Zum Erwärmen der Flüssigkeit aus den Dialysatbehältern 10 kann die Flüssigkeit dabei über den Anschluss 66 in einen Heizbeutel gepumpt werden. Dieser Heizbeutel liegt auf einem Heizelement auf, so dass die im Heizbeutel befindliche Flüssigkeit erwärmt werden kann. Daraufhin wird die Flüssigkeit aus dem Heizbeutel zum Patienten gepumpt.

Die Pumpkammer 53 und 53' und die Ventile V1 bis V4 entsprechen in Aufbau und Funktion den entsprechenden Komponenten im ersten Ausführungsbeispiel.

Im Unterschied zum ersten Ausführungsbeispiel weist die Kassette im zweiten Ausführungsbeispiel keinen Sensorbereich zum Anschluss eines Temperatursensors auf. Dieser ist vielmehr im Bereich der Heizelemente angeordnet. Die Kassette weist jedoch Messbereiche 85 und 86 zum Messen des Drucks in den Pumpekammern 53 und 53' auf. Die Messbereiche 85 und 86 sind dabei Kammern, welche mit dem Pumpkammern fluidisch in Verbindung stehen und ebenfalls von der flexiblen Folie abgedeckt werden. An die Messbereiche können geräteseitige Drucksensoren angekoppelt werden, welche den Druck in den Messkammern 85 und 86 und damit in den Pumpkammern 53 und 53' messen.

Die Verbindung der Anschlüsse 11, 21, 31 und 66 der Kassette mit den weiteren Komponenten des Fluidsystems erfolgt im zweiten Ausführungsbeispiel über Schlauchverbindungen. An diesen Schlauchverbindungen sind gegebenenfalls Konnektoren angeordnet.

### 1.3 Schläuche

Die Verbindung zwischen den einzelnen Behältern des Systems, der Kassette und dem Patientenkonnektor erfolgt üblicherweise über Schlauchverbindungen. Da es sich jeweils um Einwegartikel handelt, sind die Schläuche dabei üblicherweise zumindest auf einer Seite bereits mit einem weiteren Element fest verbunden. Z. B. können Schläuche bereits an einem oder mehreren der Anschlüsse der Kassette vorgesehen sein. Ebenfalls können Schläuche bereits fest mit Beuteln in Verbindung stehen.

### 1.4 Verbindungen

Das Fluidsystem ist üblicherweise in mehrere Teile aufgeteilt und jeweils steril verpackt. Diese Teile müssen für die Behandlung zunächst miteinander verbunden werden. Insbesondere sind dabei üblicherweise die Kassette sowie der oder die Dialysatbeutel getrennt voneinander verpackt.

Die Verbindungen zwischen den einzelnen Elementen des Fluidsystems erfolgt üblicherweise über Konnektoren. Die Konnektoren sind dabei so gestaltet, dass sie eine sterile Verbindung zwischen den einzelnen Komponenten ermöglichen. Dies erfolgt z. B. über entsprechende Schutzfolien, welche beim Schließen des Konnektors automatisch geöffnet werden.

Die Verbindung der einzelnen Komponenten kann dabei manuell durch eine Bedienperson bzw. den Patienten selbst erfolgen. Alternativ kann vorgesehen sein, dass die Verbindung der einzelnen Komponenten durch die Dialysemaschine erfolgt.

Hierzu können z. B. die entsprechenden Konnektoren in eine Konnektoraufnahme der Dialysemaschine eingelegt und automatisch durch die Dialysemaschine zusammengeführt werden.

Weiterhin kann eine elektronische Steuerung vorgesehen sein, welche überwacht, dass die richtigen Komponenten des Systems miteinander verbunden werden. Hierfür können an den Konnektoren Identifikationsmittel wie z. B. Barcodes oder RFIDs vorgesehen sein, welche die Komponenten identifizieren. Die Dialysemaschine umfasst dabei eine Identifikationsmittel-Erfassungseinheit wie z. B. einen Barcodeleser oder eine RFID-Erfassungseinheit, welcher die Identifikationsmittel auf den Konnektoren erfasst. Hierdurch kann die Steuerung der Peritonealdialyse erkennen, ob die korrekten Konnektoren eingelegt wurden.

Eine solche Überprüfung der korrekten Zusammenstellung des Fluidsystems kann dabei insbesondere mit einer automatischen Konnektion der Konnektoren kombiniert sein. Das System überprüft so zunächst, ob die richtigen Konnektoren in die Konnektorenaufnahmen eingelegt wurden. Die Verbindung zwischen den Konnektoren wird durch die Dialysemaschine nur hergestellt, wenn die richtigen Konnektoren eingelegt wurden. Andernfalls macht die Dialysemaschine den Benutzer darauf aufmerksam, dass die falschen Konnektoren eingelegt wurden.

### 2. Die Dialysemaschine

Die einzelnen Komponenten einer Dialysemaschine sollen nun im folgenden anhand von zwei Ausführungsbeispielen näher beschrieben werden.

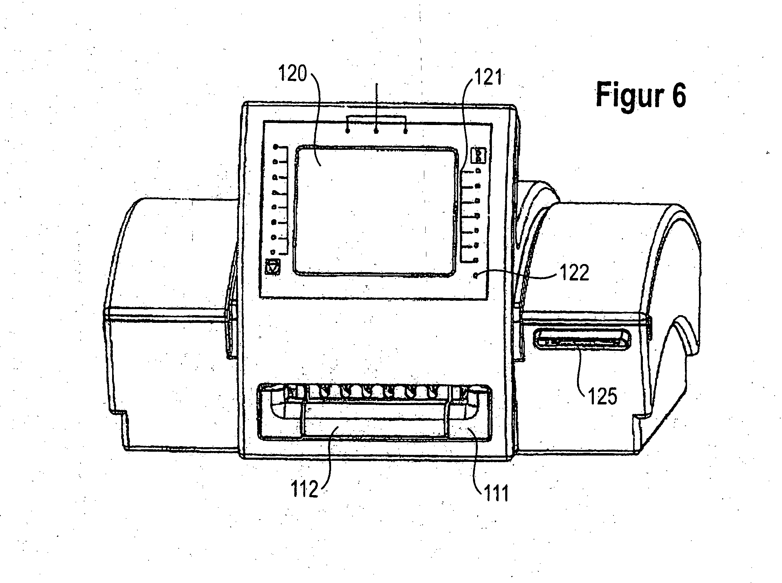

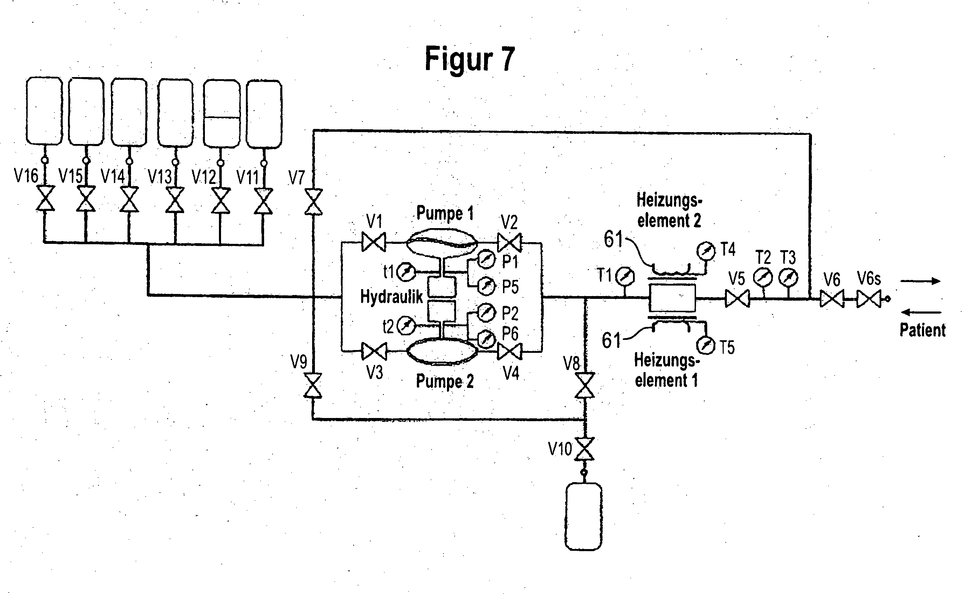

In Figur 6 ist dabei ein erstes Ausführungsbeispiel einer Dialysemaschine gezeigt, bei welchem das erste Ausführungsbeispiel einer Kassette eingesetzt wird. Das sich aus dem ersten Ausführungsbeispiel einer Dialysemaschine und dem ersten Ausführungsbeispiel einer Kassette ergebende Peritonealdialysesystem ist dabei in Figur 7 gezeigt.

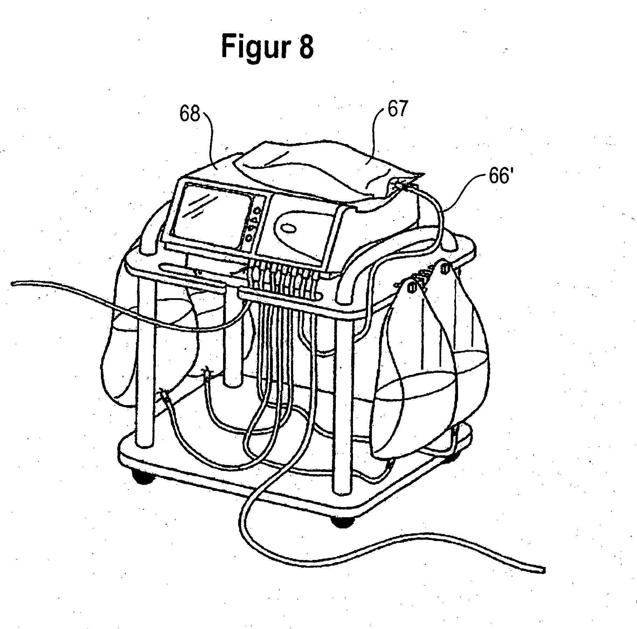

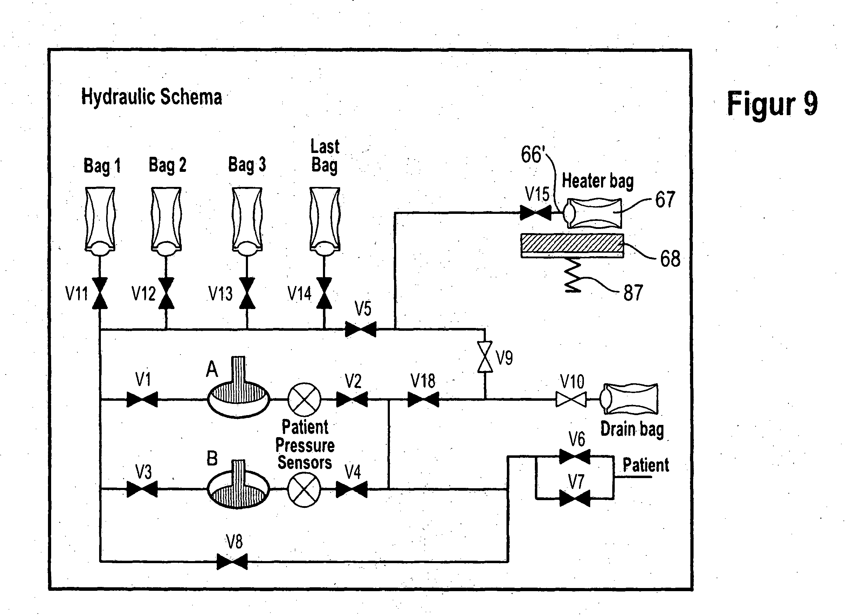

In Figur 8 ist ein zweites Ausführungsbeispiel einer Dialysemaschine, gezeigt, bei welcher das zweite Ausführungsbeispiel einer Kassette zum Einsatz kommt. Das sich aus der Kombination des zweiten Ausführungsbeispiels einer Dialysemaschine und des zweiten Ausführungsbeispiels einer Kassette ergebende Dialysesystem ist dann in Figur 9 gezeigt.

Die beiden Ausführungsbeispiele unterscheiden sich dabei zum einen in der Ausgestaltung der Heizung, in der Kopplung zwischen der Dialysemaschine und der Kassette sowie in der Ausgestaltung der Aktoren und Sensoren.

### 2.1 Heizung

Das frische Dialysat muss auf Körpertemperatur gebracht werden, bevor es in den Bauchraum des Patienten befördert wird. Hierfür weist die Dialysemaschine eine entsprechende Heizung auf.

Die Heizung erfolgt dabei üblicherweise elektrisch über ein oder mehrere Heizelemente. Bei den Heizelementen kann es sich dabei z. B. um keramische Heizelemente handeln. Bei solchen keramischen Heizelementen ist eine Widerstandsbahn auf einem Keramikträger aufgebracht. Durch Anlegen einer Spannung an die Widerstandsbahn wird diese erhitzt, wodurch sich auch das keramische Trägermaterial erhitzt. Das keramische Heizelement ist dabei üblicherweise auf einer Heizplatte angeordnet. Diese kann beispielsweise aus Aluminium gefertigt sein. An die Heizplatte werden wiederum die Fluidwege angekoppelt, so dass das in den Fluidwegen befindliche Dialysat erhitzt werden kann.

Zum Erwärmen der Flüssigkeit stehen zwei unterschiedliche Ausgestaltungen zur Verfügung. Zum einen kann zunächst eine größere Menge an Dialysat erwärmt werden, welche erst nach der Aufwärmphase zum Patienten gepumpt wird. Dies erfolgt üblicherweise über einen Heizbeutel, welcher auf einer Heizplatte des Dialysegerätes aufliegt.

Bei dem Heizbeutel kann es sich dabei um den Dialysatbeutel handeln, in welchem das Dialysat zur Verfügung gestellt wird. Üblicherweise wird jedoch ein separater Heizbeutel eingesetzt, in welchem das Dialysat zum Aufheizen hineingepumpt wird. Ist das Dialysat im Heizbeutel erwärmt, wird es von dort aus zum Patienten gepumpt.

Ein solches Konzept ist bei den in Figuren 8 und 9 gezeigten zweiten Ausführungsbeispiel einer Dialysemaschine verwirklicht. Dabei ist ein Heizbeutel 67 vorgesehen, welcher auf einer Heizplatte 68 aufliegt. Die Heizplatte 68 ist dabei auf der Oberseite des Peritonealdialysegerätes angeordnet, so dass sie leicht zugänglich ist. Der Heizbeutel 67 ist dabei über eine Leitung 66' mit der Kassette verbunden. Die Kassette weist dabei die Ventile V5, V9 und V15 auf, über welche der Heizbeutel 67 mit den übrigen Komponenten des Fluidsystems verbunden werden kann. So kann frisches Dialysat von den Dialysatbehältern 10 über die Pumpkammern zum Heizbeutel 67 gepumpt werden. Zu Beginn einer Behandlung wird also zunächst der Heizbeutel 67 mit kaltem Dialysat gefüllt. Das Dialysat im Heizbeutel 67 wird dann über die Heizplatte 68 auf Körpertemperatur erwärmt. Daraufhin wird das Dialysat über die Pumpkammern zum Patienten gepumpt. Daraufhin kann der Heizbeutel 67 wieder befüllt werden, so dass die für den nächsten Behandlungszyklus benötigte Dialysatmenge erhitzt werden kann.

Vorteilhafterweise ist dabei im Bereich der Heizplatte 68 ein Temperatursensor 88 vorgesehen, welcher mit dem Heizbeutel 67 in Kontakt steht und so die Temperatur des Dialysats im Heizbeutel 67 messen kann. Weiterhin kann ein Temperatursensor an der Heizplatte oder am Heizelement vorgesehen sein, welcher die Temperatur des Heizelements oder der Heizplatte misst. Eine entsprechende Steuerung sorgt nun dafür, dass die Heizplatte nicht zu heiß für das Material des Beutels wird.

Der Heizbeutel 67 kann zudem Funktionen bei der Bilanzierung der Flüssigkeitsströme übernehmen. So kann die Heizplatte 68 Teil einer Waage 87 sein, über welche das Gewicht des Heizbeutels 67 bestimmbar ist. Hierdurch kann die Flüssigkeitsmenge, welche nach dem Erwärmen dem Patienten zugeführt wird, bestimmt werden.

Alternativ zu der beim zweiten Ausführungsbeispiel gezeigten Erwärmung des Dialysats über einen Heizbeutel kann das Dialysat auch erwärmt werden, während es zum Patienten gepumpt wird. Die Heizung arbeitet damit in Form eines Durchlauferhitzers, welcher das durch das Fluidsystem bewegte Dialysat erwärmt, während es durch die Fluidwege gepumpt wird.

Bei diesem Konzept ist ein Dialysatkanal vorgesehen, welcher an ein Heizelement der Dialysemaschine gekoppelt wird. Während das Dialysat durch den Dialysatkanal fließt, nimmt es dabei Wärme von dem Heizelement der Dialysemaschine auf.

Ein solches Konzept ist bei dem ersten Ausführungsbeispiel einer Dialysemaschine, welches in Figuren 6 und 7 gezeigt ist, implementiert. Dabei ist der Heizbereich in die Kassette integriert, wie dies bereits oben dargestellt wurde. Beim Ankoppeln der Kassette an die Dialysemaschine kommt dabei der Heizbereich der Kassette mit Heizelementen der Dialysemaschine thermisch in Kontakt.

Die Heizelemente können dabei ebenfalls als keramische Heizelemente ausgeführt sein und mit Heizplatten in Kontakt stehen, welche dann an den Heizbereich der Kassette gekoppelt werden. Wie bereits hinsichtlich der Kassette dargestellt, steht dabei sowohl mit der Oberseite als auch mit der Unterseite des Heizbereichs jeweils eine Heizplatte in Kontakt, welche das durch den Heizbereich fließende Dialysat erwärmt.

Am Zulauf und am Ablauf des Heizbereichs sind jeweils Temperatursensorbereiche in der Kassette vorgesehen, welche durch das Ankoppeln der Kassette mit Temperatursensoren der Peritonealdialyse in Kontakt kommen. Durch die Temperatursensoren T1 bis T3 kann so die Temperatur des in den Heizbereich einfließenden Dialysats sowie die Temperatur des aus dem Heizbereich herausfließenden Dialysats bestimmt werden. Weiterhin sind Temperatursensoren T4 und T5 vorgesehen, welche die Temperatur der Heizelemente und/oder der Heizplatten bestimmen.

Die Verwendung von mindestens zwei Heizelementen ermöglicht es dabei, die Heizelemente jeweils so zusammenzuschalten, dass sie bei einer Versorgungsspannung von 220 V im wesentlichen die gleiche Leistung abgeben wird wie bei einer Versorgungsspannung von 110 V. Hierfür werden die beiden Heizelemente bei 110 V in einer Parallelschaltung betrieben, während sie bei einer Versorgungsspannung von 220 V in einer Seriellschaltung betrieben werden. Eine solche Anpassung der Verschaltung der Heizelemente an die Versorgungsspannung kann dabei unabhängig davon, ob die Heizung gemäß dem ersten oder dem zweiten Ausführungsbeispiel erfolgt, implementiert werden.

### 2.2 Ankopplung der Kassette

Um eine Ankopplung der Aktoren und/oder Sensoren der Dialysemaschine an die entsprechenden Bereiche der Kassette zu ermöglichen, weist die Dialysemaschine eine Kassettenaufnahme mit einer Ankoppelfläche auf, an welcher die Kassette angekoppelt werden kann. An der Ankoppelfläche sind die entsprechenden Aktoren, Sensoren und/oder Heizelemente der Dialysemaschine angeordnet. Die Kassette wird mit dieser Ankoppelfläche so verpresst, dass die entsprechenden Aktoren, Sensoren und/oder Heizelemente mit den entsprechenden Bereichen an der Kassette in Kontakt kommen.

Dabei ist vorteilhafterweise an der Ankoppelfläche der Dialysemaschine eine Matte aus einem flexiblen Material vorgesehen, insbesondere eine Silikonmatte. Diese sorgt dafür, dass die flexible Folie der Kassette mit den Stegbereichen der Kassette verpresst wird und so die Fluidwegen innerhalb der Kassette voneinander trennt.

Vorteilhafterweise ist weiterhin ein umlaufender Rand der Ankoppelfläche vorgesehen, welcher mit dem Randbereich der Kassette verpresst wird. Vorteilhafterweise erfolgt die Verpressung dabei luftdicht, so dass zwischen der Ankoppelfläche und der Kassette ein Unterdruck aufgebaut werden kann.

Gegebenenfalls kann auch ein Vakuumsystem vorgesehen sein, welches Luft aus dem Raum zwischen Ankoppelfläche und Kassette abpumpen kann. Hierdurch wird eine besonders gute Ankopplung der Aktoren, Sensoren und/oder Heizelementen des Peritonealdialysegerätes mit den entsprechenden Bereichen der Kassette ermöglicht. Zudem erlaubt das Vakuumsystem eine Dichtigkeitsprüfung der Kassette. Hierfür wird nach dem Ankoppeln ein entsprechendes Vakuum angelegt und überprüft, ob dieses Vakuum aufrechterhalten wird.

Das Anpressen der Kassette erfolgt z. B. pneumatisch. Hierzu ist üblicherweise ein Luftkissen vorgesehen, welches mit Druckluft befüllt wird und so die Kassette an die Ankoppelfläche anpresst.

Die Kassettenaufnahme weist üblicherweise eine der Ankoppelfläche gegenüberliegende Aufnahmefläche auf, in welche das Hartteil der Kassette eingelegt wird. Die Aufnahmefläche weist hierfür vorteilhafterweise entsprechende Vertiefungen auf. Die Aufnahmefläche mit der eingelegten Kassette kann dann über eine pneumatische Anpressvorrichtung an die Ankoppelfläche angepresst werden.

Das Einlegen der Kassette kann dabei in unterschiedlicher Weise geschehen. Im ersten Ausführungsbeispiel einer Dialysemaschine, welches in Figur 6 gezeigt ist, ist hierfür eine Schublade 111 vorgesehen, welche aus der Dialysemaschine ausgefahren werden kann. In diese Schublade wird die Kassette eingelegt. Die Kassette wird dann zusammen mit der Schublade in die Dialysemaschine eingeschoben. Daraufhin erfolgt das Verpressen der Kassette mit der Ankoppelfläche, welche im Inneren des Gerätes angeordnet ist. Dabei werden Kassette und Koppelfläche zunächst mechanisch aneinander gefahren und dann pneumatisch miteinander verpresst.

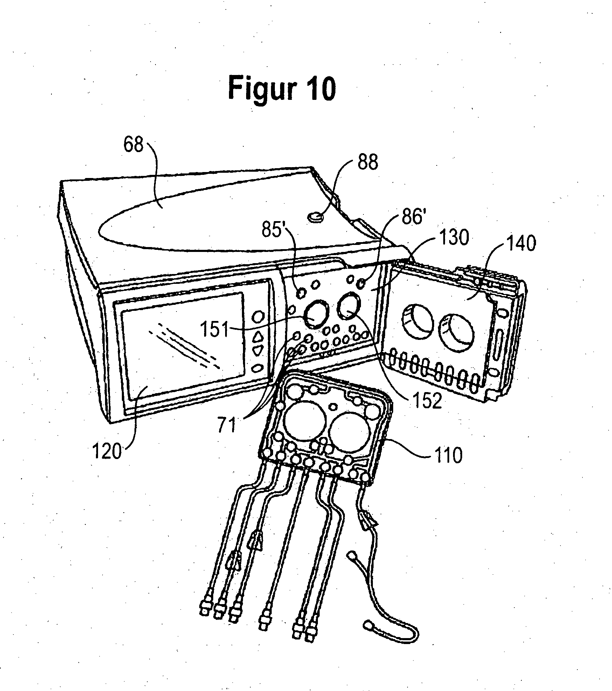

Das Ankoppeln einer Kassette 110 gemäß dem zweiten Ausführungsbeispiel ist in Figur 10 näher dargestellt. Die Ankoppelfläche 130 ist durch Öffnen einer Tür 140 frei zugänglich, so dass die Kassette in der richtigen Position an der Ankoppelfläche 130 angeordnet werden kann. Die Ankoppelfläche 130 ist dabei nach hinten zur Vertikalen geneigt, was ein leichteres Ankoppeln ermöglicht. Nun kann die Tür 140 geschlossen werden, so dass eine Aufnahmefläche an der Tür mit der Rückseite der Kassette in Kontakt kommt. Das Verpressen erfolgt nun durch ein an der Tür angeordnetes Luftkissen. Zudem wird ein Vakuum zwischen die Ankoppelfläche und die Kassette 110 angelegt.

Das erste Ausführungsbeispiel einer Dialysemaschine weist weiterhin eine Vorrichtung zum automatischen Konnektieren auf. Hierfür ist eine Konnektorenaufnahme 112 vorgesehen, in welche die Konnektoren der Dialysatbeutel 10 eingelegt werden. Die Konnektorenaufnahme 112 fährt dann in das Gerät hinein, wo ein Barcodeleser vorgesehen ist, welcher die auf den Konnektoren aufgebrachten Barcodes liest. So kann das Gerät überprüfen, ob die richtigen Beutel eingelegt wurden. Werden die richtigen Beutel erkannt, so fährt die Konnektoraufnahme 112 komplett ein und schließt so die Konnektoren der Beutel an den als Konnektoren ausgeführten Anschlüssen 11 der Kassette an.

Im zweiten Ausführungsbeispiel wurde auf eine solche automatische Konnektierung dagegen verzichtet. Daher sind an den Anschlüssen 11 der Kassette Schlauchabschnitte angeordnet, welche manuell über Konnektoren mit den entsprechenden Beuteln verbunden werden müssen.

### 2.3 Pumpaktoren

Das Pumpen der Flüssigkeit durch das Fluidsystem erfolgt in den Ausführungsbeispielen durch eine Membranpumpe, welche von den Pumpkammern 53 und 53' zusammen mit der flexiblen Folie der Kassette gebildet wird. Wird die flexible Folie dabei durch einen entsprechenden Pumpaktor in die Pumpkammer hineingedrückt, so wird Flüssigkeit aus der Pumpkammer in die geöffneten Bereiche der Fluidwege der Kassette gepumpt. Umgekehrt wird durch Herausziehen der Folie aus der Pumpkammer Fluid aus den Fluidwegen in die Pumpkammer gesaugt.

Der Pumphub erfolgt dabei durch Bewegen eines Pumpaktors in die Pumpkammer. Für den Saughub wird der Pumpaktor wieder von der Pumpkammer wegbewegt. Durch die luftdichte Verpressung von Kassette und Ankoppelfläche entsteht dabei ein Unterdruck, durch welchen die flexible Folie der Kassette dem Pumpaktor folgt und so wieder aus der Pumpkammer herausgezogen wird.

Um eine gute Ankopplung des Pumpaktors an die flexible Folie der Kassette zu ermöglichen, kann zudem ein Vakuumsystem vorgesehen sein. Über das Einstellen eines entsprechenden Vakuums zwischen der Ankoppelfläche und der Kassette kann dabei insbesondere die Kraft eingestellt werden, mit welcher die flexible Folie während eines Saughubs maximal von der Pumpkammer wegbewegt wird.

Hierdurch kann die Saugkraft der Pumpe sehr fein eingestellt werden. Die Pumpkraft wird dagegen durch die Schubkraft des Aktors eingestellt.

Die Bilanzierung der Flüssigkeitsströme kann dabei durch das Zählen der Saug- und Pumphübe erfolgen, da die Membranpumpe eine hohe Genauigkeit der mit jedem Hub gepumpten Flüssigkeitsmenge aufweist.

### 2.3.1. Hydraulischer Antrieb

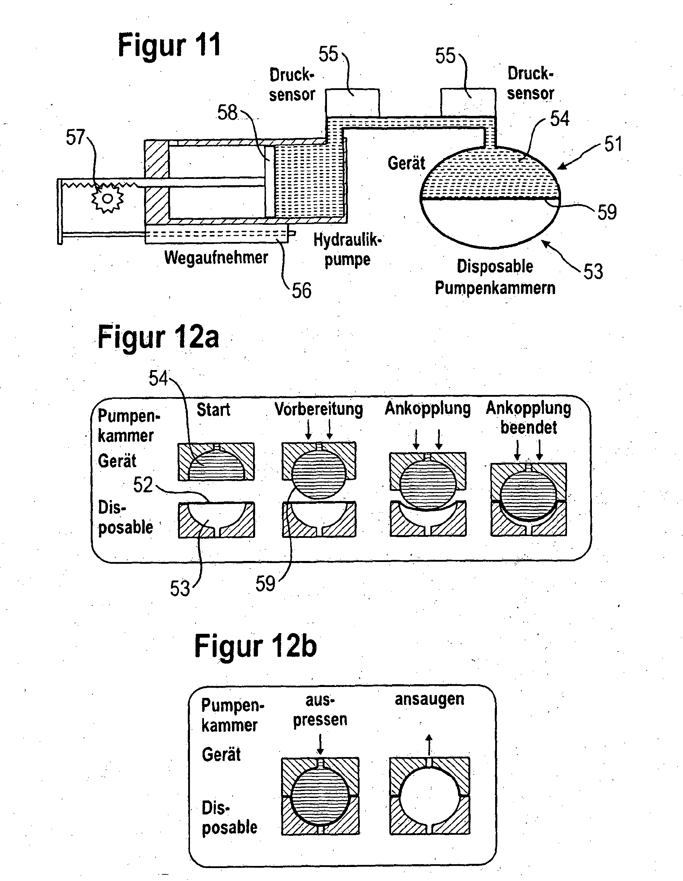

Der Aufbau eines ersten Ausführungsbeispiels eines Pumpaktors ist in Figur 11 gezeigt. Der Pumpaktor wird dabei hydraulisch bewegt. Hierfür ist eine Membran 59 vorgesehen, welche an der flexiblen Folie der Kassette anlegt. Die Membran 59 kann dabei z. B. aus Silikon gefertigt sein. Hinter der Membran 59 ist eine Kammer 54 vorgesehen, welche mit Hydraulikfluid gefüllt werden kann. Durch Anlegen eines Überdrucks in der Kammer 54 wird die Membran 59 und mit dieser die flexible Folie in die Pumpkammer 53 der Kassette hineingedrückt. Durch Anlegen eines Unterdruckes an die Kammer 54 wird die Membran 59 dagegen in die Kammer 54 hineingezogen. Durch den Unterdruck zwischen der flexiblen Folie und der Membran folgt die flexible Folie dieser Bewegung, so dass sich das Volumen der Pumpkammer 53 vergrößert. Der Pumpvorgang mit den Pumphub und dem Ansaughub ist dabei schematisch in Figur 12b dargestellt.

Zum Betrieb der Pumphydraulik ist eine Hydraulikpumpe 58 vorgesehen. Diese weist einen Zylinder auf, in welchem ein Kolben über einen Motor 57 hin und her bewegt werden kann. Hierdurch wird die Hydraulikflüssigkeit über eine entsprechende Verbindungsleitung in die Kammer 54 hineingepreßt oder aus dieser wieder herausgesogen. An der Hydraulikpumpe 58 ist dabei ein Wegaufnehmer 56 vorgesehen, über welchen die Bewegung des Kolbens aufgenommen werden kann. Hierdurch kann bestimmt werden, wieviel Hydraulikflüssigkeit in die Kammer 54 hineingepreßt bzw. wieviel Hydraulikflüssigkeit aus dieser entnommen wurde. Weiterhin sind Drucksensoren 55 an der Hydraulik vorgesehen, welche den Druck in dem Hydrauliksystem messen. Diese ermöglichen zum einen eine Funktionsüberprüfung der Hydraulik, da die Daten der Drucksensoren mit denen des Wegaufnehmers 56 verglichen werden können und hierdurch die Dichtigkeit des Hydrauliksystems überprüft werden kann.

Zudem ermöglichen die Drucksensoren eine Bestimmung des Drucks in der Pumpekammer 53 der Kassette. Wird die Hydraulikpumpe 58 nicht bewegt, so stellt sich ein Druckgleichgewicht zwischen der Kammer 54 und der Pumpkammer 53 ein. Der Druck der Hydraulikflüssigkeit entspricht damit dem Druck in der Pumpenkammer 53.

In Figur 12a ist nun der Ankoppelvorgang des Pumpenaktors an die Pumpenkammer 53 gezeigt. Zur Vorbereitung des Ankoppeln wird dabei zunächst die Kammer 54 mit Hydraulikfluid beaufschlagt, dass sich die Membran 59 nach außen wölbt. Daraufhin werden Ankoppelfläche und Kassette aufeinander zu bewegt, so dass die Membran 59 die flexible Folie der Kassette in die Pumpkammer 53 hineindrückt. Nach dem Verpressen von Ankoppelfläche und der Kassette ist der Raum zwischen der Membran und der flexiblen Folie nach außen luftdicht abgeschlossen, so dass die flexible Folie der Bewegung der Membran folgt. Dies ist in Figur 12b gezeigt.

Der in Figur 11 gezeigte Pumpaktor ist dabei beim ersten Ausführungsbeispiel einer Dialysemaschine implementiert, wie dies auch aus Figur 7 ersichtlich ist. Dabei ist für jede der beiden Pumpkammern 53 und 53' jeweils ein entsprechender Pumpaktor vorgesehen.

### 2.3.2 Elektromechanischer Antrieb

Alternativ kann der Pumpaktor auch elektromotorisch betrieben werden. Hierfür ist ein entsprechend geformter Stempel vorgesehen, welcher über einen Elektromotor, insbesondere über einen Schrittmotor gegen die flexible Folie gedrückt bzw. von dieser wegbewegt wird und so den Pump- bzw. Saughub erzeugt. Solche Pumpaktoren 151 und 152 sind bei dem Ausführungsbeispiel in Fig. 10 gezeigt. Vorteilhafterweise ist dabei ein Vakuumsystem vorgesehen, welches dafür sorgt, dass die flexible Folie dem Stempel auch bei der Saugbewegung folgt.

### 2.4 Ventilaktoren

Als Ventilaktor kann ein Ventilstößel vorgesehen sein, welcher die flexible Folie der Kassette in eine entsprechende Kammer des Hartteils hineindrückt und so den Fluidkanal in diesem Bereich schließt. Der Ventilaktor kann dabei Z.B. pneumatisch betätigt werden. Der Stößel kann dabei über eine Feder vorgespannt sein, so dass er entweder drucklos öffnet oder drucklos schließt.

Alternativ kann der Ventilaktor über eine flexible Membran implementiert werden, welche hydraulisch oder pneumatisch bewegt wird. Die flexible Membran wird dabei durch Anlegen von Druck gegen die Kassette bewegt und drückt so einen entsprechenden Ventilbereich der flexiblen Folie in einen Fluidkanal, um diesen zu verschließen.

Ventilaktoren 71, welche an die Ventilbereiche V1 bis V16 der Kassette angekoppelt werden, sind in Fig. 10 auf der Ankoppelfläche erkennbar.

### 2.5 Sensoren

Die Dialysemaschine weist Sensoren auf, über welche die Maschine angesteuert bzw. deren ordnungsgemäßes Funktionieren überwacht werden kann.

Zum einen sind dabei ein oder mehrere Temperatursensoren vorgesehen, über welche die Temperatur des Dialysats und/oder der Heizelemente gemessen werden kann. Im ersten Ausführungsbeispiel sind die Temperatursensoren dabei an der Ankoppelfläche zur Kassette angeordnet und können so die Temperatur des durch die Kassette fließenden Dialysats messen. Im zweiten Ausführungsbeispiel ist dagegen ein Temperatursensor 88 auf der Heizplatte 68 vorgesehen, welcher die Temperatur des im Beutel 67 befindlichen Dialysats mißt. Weiterhin können an dem oder den Heizelementen Temperatursensoren vorgesehen sein.

Weiterhin können ein oder mehrere Drucksensoren vorgesehen sein, um den Druck in den Pumpkammern zu bestimmen. Hierdurch kann verhindert werden, das Dialysat mit zu hohem Druck zum Patienten gepumpt wird oder der Saugdruck beim Absaugen von Dialysat vom Patienten zu hoch wird.

Im ersten Ausführungsbeispiel erfolgt die Druckmessung dabei über Drucksensoren in der Hydraulik der Pumpaktoren, wie dies oben dargestellt wurde. Im zweiten Ausführungsbeispiel sind dagegen Drucksensoren 85' und 86' in der Ankoppelfläche vorgesehen, welche den Druck in entsprechenden Druckmessbereichen der Kassette direkt messen. Die Ankopplung dieser Drucksensoren an die Kassette wird dabei vorteilhafterweise durch ein Vakuumsystem sichergestellt.

### 2.6 Ein-/Ausgabeeinheit

Die Dialysemaschine umfasst weiterhin eine Ein-/Ausgabeeinheit zur Kommunikation mit einem Bediener. Zur Ausgabe von Informationen ist dabei eine entsprechende Anzeige vorgesehen, welche z. B. durch Leuchtdioden, LCD-Anzeigen oder einen Bildschirm implementiert sein kann. Zur Eingabe von Befehlen sind entsprechende Eingabeelemente vorgesehen. Hierbei können z. B. Drucktasten und Schalter vorgesehen sein.

In beiden Ausführungsbeispielen ist dabei ein Touchscreen 120 vorgesehen, welcher eine interaktive Menüführung ermöglicht. Weiterhin sind Anzeigeelemente 121 und 122 vorgesehen, welche Zustände der Dialysemaschine kompakt darstellen.

Das erste Ausführungsbeispiel weist weiterhin einen Kartenleser 125 auf, über welchen eine Patientenkarte eingelesen werden kann. Auf der Patientenkarte können Daten zur Behandlung des jeweiligen Patienten abgespeichert sein. Hierdurch kann der Behandlungsablauf für den jeweiligen Patienten individuell festgelegt werden.

Die Peritonealdialyse weist weiterhin eine akustische Signaleinheit auf, über welche akustische Signale abgegeben werden können. Insbesondere kann dabei ein akustisches Warnsignal ausgegeben werden, wenn ein Fehlzustand registriert wird. Dabei ist vorteilhafterweise ein Lautsprecher vorgesehen, über welchen die akustischen Signale erzeugt werden können.

### 2.7 Steuerung

Die Peritonealdialyse weist weiterhin eine Steuerung auf, durch welche sämtliche Komponenten angesteuert und überwacht werden. Die Steuerung sorgt dabei für den automatischen Ablauf der Behandlung.

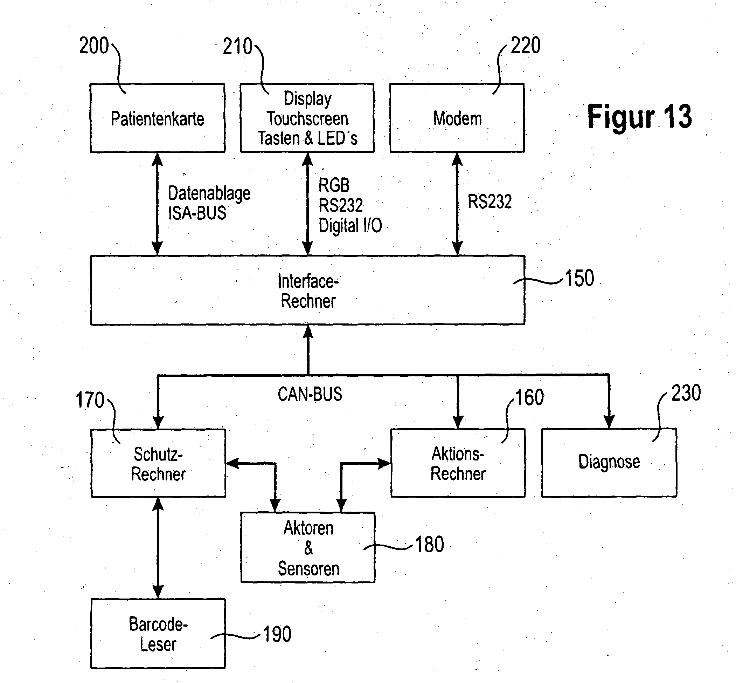

In Figur 13 ist nun der prinzipielle Aufbau eines Ausführungsbeispiels einer solchen Steuerung dargestellt.

Die Kommunikation mit dem Bediener sowie mit externen Informationsquellen erfolgt dabei über einen Interface-Rechner 150. Dieser kommuniziert mit einem Patientenkartenleser 200, einer Ein- und Ausgabeeinheit 210, welche der Kommunikation mit dem Patienten dient, sowie mit einem Modem 220. Über das Modem 220 kann z. B. eine aktualisierte Software aufgespielt werden.

Der Interface-Rechner 150 steht über einen internen Bus mit einem Aktionsrechner 160 und einem Schutzrechner 170 in Verbindung. Der Aktionsrechner 160 und der Schutzrechner 170 erzeugen eine Redundanz des Systems. Der Aktionsrechner 160 erhält dabei Signale von den Sensoren des Systems und berechnet die Steuersignale für die Aktoren 180. Der Schutzrechner 170 erhält ebenfalls Signale von den Sensoren 180 und überprüft, ob die vom Aktionsrechner 160 ausgegebenen Befehle korrekt sind. Stellt der Schutzrechner 170 einen Fehler fest, so leitet er eine entsprechende Notfallprozedur ein. Insbesondere kann der Schutzrechner 170 dabei ein Alarmsignal auslösen. Weiterhin kann der Schutzrechner 170 den Zugang zum Patienten schließen. Hierfür ist ein spezielles Ventil am patientenseitigen Ausgang der Kassette angeordnet, auf welches nur der Schutzrechner 170 Zugriff hat. Dieses Sicherheitsventil ist dabei im drucklosen Zustand geschlossen, so dass es bei einem Ausfall der Pneumatik automatisch schließt.

Der Schutzrechner 170 steht weiterhin mit dem Barcodeleser 190 in Verbindung, und überprüft so den Anschluss der korrekten Dialysatbeutel.

Weiterhin ist ein Diagnosesystem 230 vorgesehen, über welches Fehler des Systems ermittelt und behoben werden können.

### 3. Implementierung der Erfindung

Ein Ausführungsbeispiel der vorliegenden Erfindung, welches bei einem oben dargestellten Dialysesystemen bzw. bei einer der oben dargestellten Dialysemaschinen zum Einsatz kommt, wird nun im folgenden dargestellt. Dabei kann das Ausführungsbeispiel der vorliegenden Erfindung mit einzelnen oder mehreren Komponenten, wie sie oben beschrieben wurden, kombiniert werden.

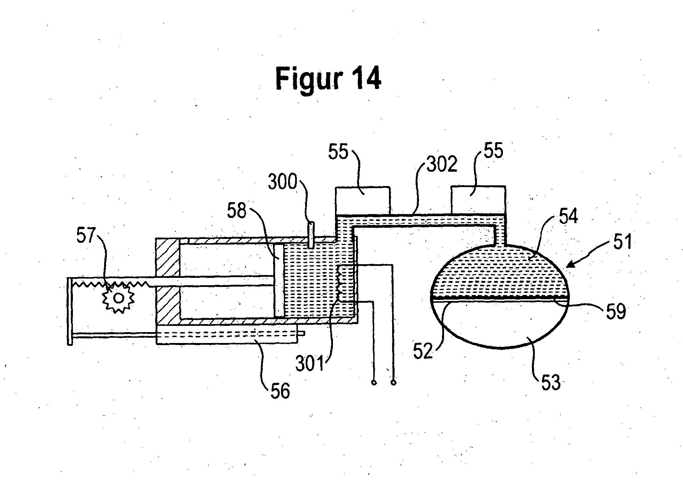

In Figur 14 ist nun ein erstes Ausführungsbeispiel eines beheizbaren Aktors 51 gemäß der vorliegenden Erfindung dargestellt. Der Aktor 51 ist dabei an einem Aktorbereich 52 des Fluidsystems ankoppelbar. Bei dem Aktorbereich 52 handelt es sich dabei um eine Pumpmembran, welche zum Pumpen von Flüssigkeit innerhalb des Fluidsystems bewegt werden kann.

Die Pumpmembran ist dabei als flexible Folie ausgeführt, welche an einer Pumpkammer 53 einer Kassette angeordnet ist. Der weitere Aufbau der Kassette und der Pumpkammer wurde bereits oben näher dargestellt.

An die Pumpmembran des Fluidsystems ist eine flexible Membran 59 des Aktors ankoppelbar. Das Ankoppeln erfolgt dabei vorteilhafterweise durch Verpressen von Ankoppelfläche und Kassette. Gegebenenfalls kann dabei zusätzlich ein Vakuum zwischen Ankoppelfläche und Kassette eingebracht werden.

Die flexible Membran 59 des Aktors ist hydraulisch betätigbar, indem Hydraulikfluid in die Hydraulikkammer 54 hineingepumpt oder aus dieser herausgepumpt wird. Die Funktion des Pumpaktors wurde dabei bereits oben näher dargestellt.

Erfindungsgemäß ist nun ein Heizelement 301 vorgesehen, über welches die Hydraulikflüssigkeit zum Bewegen des Pumpaktors 51 erwärmt werden kann. Das Heizelement 301 ist dabei im Ausführungsbeispiel im Bereich der Pumpe 58 angeordnet. Alternativ zur in Figur 14 gezeigten Anordnung des Heizelements 301 im Bereich der Pumpe könnte dieses auch in der Hydraulikleitung 302 zwischen der Hydraulikpumpe und der Hydraulikkammer 54 angeordnet werden.

Weiterhin ist ein Temperatursensor 300 vorgesehen, über dessen Ausgangssignal das Heizelement 301 angesteuert werden kann. Der Temperatursensor 300 misst dabei die Temperatur des Hydraulikfluids, so dass die Temperatur des Hydraulikfluids geregelt werden kann. Bei einer Pumpbewegung der Hydraulikpumpe 58 wird das über das Heizelement 301 erwärmte Hydraulikfluid zur flexiblen Membran 59 des Aktors bewegt. Dort kann das erwärmte Hydraulikfluid die Flüssigkeit in der Pumpkammer 53 des Fluidsystems erwärmen.

Bei dem Heizelement 301 handelt es sich um ein elektrisches Heizelement. Insbesondere handelt es sich dabei um eine Widerstandsstrecke, an welche eine elektrische Spannung angelegt wird. Vorteilhafterweise handelt es sich dabei um ein keramisches Heizelement, bei welchem die Widerstandsstrecke auf einem keramischen Trägermaterial aufgebracht ist. Das erfindungsgemäße Heizelement kann dabei eine Heizleistung zwischen 5 W und 1000 W aufweisen. Weiterhin vorteilhafterweise weist das Heizelement dabei eine Heizleistung von mehr als 50 W auf.

Das Heizelement 301 ist gegenüber dem Hydraulikfluid elektrisch isoliert. Insbesondere kann es sich dabei um einen Heizstab oder um eine Heizplatte handeln, welche elektrisch von dem Hydraulikfluid isoliert ist. Weiterhin ist auch die flexible Membran 59 elektrisch isolierend ausgeführt. Hierdurch ergibt sich eine doppelte Isolierung zwischen dem Heizelement 301 und der Kassette. Hierdurch kann die Dialysemaschine gegebenenfalls ohne Schutzkontakt ausgeführt werden und dennoch die Schutzklasse 2 aufweisen.

Weiterhin sind im Ausführungsbeispiel zwei Aktoren vorgesehen, welche zur Erwärmung der Flüssigkeit im Fluidsystem herangezogen werden. Insbesondere handelt es sich dabei um die beiden Pumpaktoren, welche jeweils auf die Pumpbereiche 53 und 53' einwirken. Dabei kann entweder ein gemeinsames Heizelement zum Beheizen der beiden Aktoren eingesetzt werden. Alternativ kann auch jeder der beiden Aktoren über ein eigenes Heizelement beheizt werden. In diesem Fall ist vorteilhafterweise jeder der beiden Aktoren mit einem Temperatursensor ausgestattet.

Vorteilhafterweise umfasst die Dialysemaschine ein weiteres Heizelement zum Erwärmen von Flüssigkeit im Fluidsystem, wie dies z.B. oben näher dargestellt wurde. Vorteilhafterweise ist das Heizelement 301 dabei in die Temperatursteuerung der Dialysemaschine integriert. Insbesondere kann dabei der erfindungsgemäß beheizbare Pumpaktor zum Vorheizen der Flüssigkeit im Fluidsystem eingesetzt werden.

Der erfindungsgemäße Aktor kann dabei bei einer Peritonealdialysemaschine eingesetzt werden, wie sie oben dargestellt wurde. Insbesondere sind dabei einer oder mehrere der erfindungsgemäßen Aktoren an der Ankoppelfläche der Peritonealdialysemaschine eingesetzt. Dabei können die Pumpaktoren und/oder die Ventilaktoren beheizbar ausgeführt sein.

Ebenso kann die vorliegende Erfindung aber auch bei der Hämodialyse zum Einsatz kommen. Insbesondere kann bei der Hämodialyse eine Kassette, wie sie oben für die Peritonealdialyse dargestellt wurde, in gleicher Weise eingesetzt werden. Dabei kann ein Aktor eingesetzt werden, um eine in einem Aktorbereich der Kassette befindliche Flüssigkeit zu erwärmen.

Dabei können die gleichen Komponenten, wie sie oben hinsichtlich der Peritonealdialyse beschrieben wurden, auch für die Hämodialyse und damit für eine Hämodialysemaschine eingesetzt werden. Das Fluidsystem kann dabei sowohl zum Transport von Dialysat, als auch zum Transport von Blut eingesetzt werden. Demgemäß kann der erfindungsgemäße Aktor zum Erwärmen einer medizinischen Flüssigkeit, insbesondere von Dialysat oder von Blut, eingesetzt werden.

Die vorliegenden Erfindung ermöglicht durch die Nutzung des Aktorbereichs zum Erwärmen einer Flüssigkeit im Fluidsystem einen besonders kompakten Aufbau des Fluidsystems, insbesondere der Kassette.

### Bezugszeichenliste

Behandlungszyklen 1
   Einlaufphase 2
   Basiseinlaufphase 2'
   Verweilphase 3
   Auslaufphase 4
   Basisauslaufphase 4'
   initialer Auslauf 5
   Auslaufphase 5'
   letzter Einlauf 6
   Einlaufphase 6'
   Basiszyklus 7
   Tidalzyklen 8
   Nacht 9
Behälter 10
   Anschlüsse 11 zum Anschluss von Behältern 10
Abfluss 20
   Anschluss 21 zum Anschluss an den Abfluss 20
Konnektor 30
   Anschluss 31 zum Anschluss an den Konnektor 30
Dialysemaschine 40
Pumpe 50
   Pumpaktoren 51
   Pumpbereich 52
   Pumpenkammern 53 und 53'
   (Hydraulik)Kammer 54
   Drucksensoren 55
   Wegaufnehmer 56
   Motor 57
   Hydraulikpumpe 58
   Membran 59
Heizung 60
   Heizelement 61
   Heizbereich 62
   Unterseite des Heizbereichs 63
   Stege 64
   Durchbruch 65
   Anschluss 66 für einen Heizbeutel 67
   Leitung 66'
   Heizbeutel 67
   Heizplatte 68
Ventile 70
   Ventilaktoren 71
   Ventilbereiche 72
   Ventilbereiche 73 für die Pumpkammern
   Ventilbereiche V1 bis V16
Sensoren 80
   Sensoren 81
   Sensorbereiche 82
   Sensorbereiche 83 und 84 der Temperatursensoren
   Sensorbereiche 85 und 86 der Drucksensoren
   Drucksensoren 85'und 86'
   Waage 87
   Temperatursensor 88
   Temperatursensoren T1 bis T3
   Temperatursensoren T4 und T5
Steuerung 90
   Bilanzierung 95
Fluidwege 100
   Hartteil 101
   flexible Folie 102
   Kassette 110
Schublade 111
   Konnektorenaufnahme 112
   Touchscreen 120
   Anzeigeelemente 121 und 122
   Kartenleser 125
   Ankoppelfläche 130
   Tür 140
   Pumpaktoren 151 und 152
Interface-Rechner 150.
   Aktionsrechner 160
   Schutzrechner 170
   Aktoren und Sensoren 180
   Barcodeleser 190
   Patientenkartenleser 200
   Ein- und Ausgabeeinheit 210
   Modem 220
   Diagnosesystem 230
Temperatursensor 300
   Heizelement 301
   Hydraulikleitung 302

## Patentansprüche

1. Dialysemaschine mit einer Ankoppelfläche (130) zum Ankoppeln eines Fluidsystems, insbesondere zum Ankoppeln einer Kassette (110), und einem auf der Ankoppelfläche (130) angeordneten Aktor zum Bewegen eines Aktor-Bereichs des Fluidsystems,
**gekennzeichnet durch** ein Heizlement (301) zum Beheizen des Aktors, welcher hydraulisch betätigbar ist und wobei die Flüssigkeit im Aktor-Bereich des Fluidsystems über den Aktor erwärmbar ist, und wobei das Heizelement (301) in einer Hydraulikpumpe (58) oder in einer Hydraulikleitung (302) angeordnet ist.

2. Dialysemaschine (40) nach Anspruch 1, **gekennzeichnet durch** einen Temperatursensor (300) zum Bestimmen der Temperatur des Aktors und/oder des Heizelements (301).

3. Dialysemaschine (40) nach Anspruch 1, **gekennzeichnet durch** eine Heizungssteuerung zur Ansteuerung des Heizelements (301), insbesondere auf Grundlage des Ausgangssignals eines Temperatursensors (300).

4. Dialysemaschine (40) nach einem der vorhergehenden Ansprüche, **dadurch gekennzeichnet, dass** das Heizlement (301) vorgesehen ist, um die Hydraulikflüssigkeit zum Antrieb des Aktors zu erwärmen.

5. Dialysemaschine (40) nach Anspruch 4, **dadurch gekennzeichnet, dass** der Aktor eine flexible Membran (59), welche durch das Hydraulikfluid bewegbar ist, aufweist.

6. Dialysemaschine (40) nach Anspruch 5, **dadurch gekennzeichnet, dass** die flexible Membran (59) an dem Aktor-Bereich der Fluidwege anliegt.

7. Dialysemaschine (40) nach einem der Ansprüche 4 bis 6, **dadurch gekennzeichnet, dass** das Heizlement (301) elektrisch von der Hydraulikflüssigkeit isoliert ist.

8. Dialysemaschine (40) nach einem der Ansprüche 5 bis 8, **dadurch gekennzeichnet, dass** die flexible Membran (59) elektrisch isolierend ausgeführt ist.

9. Dialysemaschine (40) nach einem der Ansprüche 4 bis 8, **dadurch gekennzeichnet, dass** ein Temperatursensor (300) zur Bestimmung der Temperatur des Hydraulikfluids vorgesehen ist.

10. Dialysemaschine (40) nach einem der vorhergehenden Ansprüche, **dadurch gekennzeichnet, dass** es sich bei dem Aktor um einen Pump-Aktor (51) zum Bewegen einer Pump-Membran (59) des Fluidsystems handelt.

11. Dialysemaschine (40) nach einem der vorhergehenden Ansprüche, **dadurch gekennzeichnet, dass** mindestens ein weiteres Heizlement (301) vorgesehen ist, welches mit einem unbewegten Heizbereich des Fluidsystems koppelbar ist.

12. Dialysemaschine (40) nach Anspruch 11, **dadurch gekennzeichnet, dass** der Aktor derart angeordnet ist, das zum Heizbereich fließende Fluid vorzuheizen.

13. System aus einer Dialysemaschine (40) nach einem der vorhergehenden Ansprüche und einem Fluidsystem, insbesondere einer Kassette (110).

14. Verfahren zum Betrieb einer Dialysemaschine (40), mit den Schritten:
- Ankoppeln eines Fluidsystems, insbesondere einer Kassette (110), an einer Ankoppelfläche (130) der Dialysemaschine (40),
- Bewegen eines Aktor-Bereichs des Fluidsystems durch einen auf der Ankoppelfläche (130) angeordneten hydraulisch betätigbaren Aktor, wobei ein Heizelement (301) zum Beheizen des Aktors in einer Hydraulikpumpe (58) oder in einer Hydraulikleitung (302) angeordnet ist, und
- Erwärmen einer im Aktor-Bereich des Fluidsystems befindlichen Flüssigkeit über den Aktor.

## Claims

1. A dialysis machine comprising a coupling surface (130) for the coupling of a fluid system, in particular for the coupling of a cassette (110); and an actuator arranged on the coupling surface (130) for the movement of an actuator region of the fluid system,
**characterized by** a heating element (301) for the heating of the actuator which can be actuated hydraulically, with the liquid in the actuator region of the fluid system being heatable via the actuator and with the heating element (301) being arranged in a hydraulic pump (58) or in a hydraulic line (302).

2. A dialysis machine (40) in accordance with claim 1 **characterized by** a temperature sensor (300) for the determination of the temperature of the actuator and/or of the heating element (301).

3. A dialysis machine (40) in accordance with claim 1 **characterized by** a heating controller for the control of the heating element (301), in particular on the basis of the output signal of a temperature sensor (300).

4. A dialysis machine (40) in accordance with one of the preceding claims, wherein the heating element (301) is provided to heat the hydraulic fluid for the drive of the actuator.

5. A dialysis machine (40) in accordance with claim 4, wherein the actuator has a flexible membrane (59) which can be moved by the hydraulic fluid.

6. A dialysis machine (40) in accordance with claim 5, wherein the flexible membrane (59) contacts the actuator region of the fluid paths.

7. A dialysis machine (40) in accordance with one of the claims 4 to 6, wherein the heating element (301) is electrically insulated from the hydraulic fluid.

8. A dialysis machine (40) in accordance with one of claims 5 to 8, wherein the flexible membrane (59) is made as electrically insulating.

9. A dialysis machine (40) in accordance with one of the claims 4 to 8, wherein a temperature sensor (300) is provided for the determination of the temperature of the hydraulic fluid.

10. A dialysis machine (40) in accordance with one of the preceding claims, wherein the actuator is a pump actuator (51) for the movement of a pump membrane (59) of the fluid system.

11. A dialysis machine (40) in accordance with one of the preceding claims, wherein at least one further heating element (301) is provided which can be coupled to an unmoved heating region of the fluid system.

12. A dialysis machine (40) in accordance with claim 11, wherein the actuator is arranged in such a manner as to preheat the fluid flowing to the heating region.

13. A system of a dialysis machine (40) in accordance with one of the preceding claims and of a fluid system, in particular of a cassette (110).

14. A method for the operation of a dialysis machine (40), comprising the steps:
- coupling a fluid system, in particular a cassette (110), to a coupling surface (130) of the dialysis machine (40);
- moving an actuator region of the fluid system by an actuator which can be actuated hydraulically and which is arranged on the coupling surface (130), with a heating element (301) for heating the actuator being arranged in a hydraulic pump (58) or in a hydraulic line (302);
- heating a liquid present in the actuator region of the fluid system via the actuator.

## Revendications

1. Machine de dialyse comprenant une surface de couplage (130) destinée au couplage d'un système de fluide, en particulier au couplage d'une cassette (110), et un actionneur disposé sur la surface de couplage (130) destiné à mouvoir une zone d'actionnement du système de fluide,
**caractérisée par** un élément chauffant (301) destiné à chauffer l'actionneur, qui est à commande hydraulique, le liquide dans la zone d'actionnement du système de fluide pouvant être chauffé par le biais de l'actionneur, et l'élément chauffant (301) étant disposé dans une pompe hydraulique (58) ou dans un conduit hydraulique (302).

2. Machine de dialyse (40) selon la revendication 1, **caractérisée par** un capteur de température (300) destiné à déterminer la température de l'actionneur et/ou de l'élément chauffant (301).

3. Machine de dialyse (40) selon la revendication 1, **caractérisée par** une commande de chauffage destinée à commander l'élément chauffant (301), en particulier sur la base du signal de sortie d'un capteur de température (300).

4. Machine de dialyse (40) selon l'une des revendications précédentes, **caractérisée en ce que** l'élément chauffant (301) est prévu pour chauffer le liquide hydraulique pour l'entraînement de l'actionneur.

5. Machine de dialyse (40) selon la revendication 4, **caractérisée en ce que** l'actionneur comporte une membrane flexible (59), qui peut être mue par le fluide hydraulique.

6. Machine de dialyse (40) selon la revendication 5, **caractérisée en ce que** la membrane flexible (59) est adjacente à la zone d'actionnement des voies d'écoulement de fluide.

7. Machine de dialyse (40) selon l'une des revendications 4 à 6, **caractérisée en ce que** l'élément chauffant (301) est isolé électriquement du liquide hydraulique.

8. Machine de dialyse (40) selon l'une des revendications 5 à 8, **caractérisée en ce que** la membrane flexible (59) est réalisée isolante du point de vue électrique.

9. Machine de dialyse (40) selon l'une des revendications 4 à 8, **caractérisée en ce qu'**un capteur de température (300) est prévu pour déterminer la température du fluide hydraulique.

10. Machine de dialyse (40) selon l'une des revendications précédentes, **caractérisée en ce que** l'actionneur est un actionneur de pompe (51) destiné à mouvoir une membrane de pompe (59) du système de fluide.

11. Machine de dialyse (40) selon l'une des revendications précédentes, **caractérisée en ce qu'**au moins un autre élément chauffant (301) qui peut être couplé avec une zone chauffante immobile du système de fluide est prévu.

12. Machine de dialyse (40) selon la revendication 11, **caractérisée en ce que** l'actionneur est disposé de façon à préchauffer le fluide s'écoulant vers la zone chauffante.

13. Système constitué d'une machine de dialyse (40) selon l'une des revendications précédentes et d'un système de fluide, en particulier d'une cassette (110).

14. Procédé de fonctionnement d'une machine de dialyse (40), comprenant les étapes consistant à :
- coupler un système de fluide, en particulier une cassette (110), sur une surface de couplage (130) de la machine de dialyse (40),
- mouvoir une zone d'actionnement du système de fluide au moyen d'un actionneur à commande hydraulique disposé sur la surface de couplage (130), un élément chauffant (301) destiné à chauffer l'actionneur étant disposé dans une pompe hydraulique (58) ou dans un conduit hydraulique (302), et
- chauffer un liquide se trouvant dans la zone d'actionnement du système de fluide par le biais de l'actionneur.
